# EUROPEAN PATENT APPLICATION

(11) **EP 2 050 828 A2**
(43) Date of publication of application: **22.04.2009**
(21) Application number: 09000816.0
(22) Date of filing: 12.06.2007
(51) Int. Cl.: C12Q 1/68

(54) **Methylation markers for early detection and prognosis of colon cancers**

(30) Priority: 12.06.2006 US 812635 P
(62) Divisional of application: 07809489.3
(71) Applicant: Oncomethylome Sciences S.A., Durham, NC 27713 (US)
(72) Inventor: Straub, Jozef, 3001 Leuven (BE); Van Criekenge, Wim, 3001 Leuven (BE); De Carvalho, Beatriz Pinto Morais, 3001 Leuven (BE); Meijer, Gerritt, 3001 Leuven (BE)
(74) Representative: Tombling, Adrian George

(57) **Abstract**

Using a combination of analytic methods epigenetic silencing of markers in cancer have been determined. The cancers are generally those of the gastrointestinal tract including but not limited to esophageal, head and neck, gastric, pancreas, liver and colon. The genes can be used for the early detection of cancer or can be used to identify adenomas that will likely progress to carcinomas. Therapeutic regimens based on the epigenetic silenced genes can be chosen and/or monitored. Kits for evaluating epigenetic silencing of these genes can be used for detection and monitoring.

## Description

This application claims the benefit of U.S. Provisional Application S.N. 60/812,635 filed June 12, 2006, the entire contents of which are incorporated by reference herein.

The accompanying compact disk (submitted in three copies) is incorporated herein by reference. The files contained on the disk are: (1) splices.txt, 3 kb, created June 8, 2006; (2) prot.fasta, 151 kb, created June 8, 2006; (3) prom 5000-1000.fasta, 1596 kb, created June 8, 2006; (4) mma.fasta, 854 kb, created June 8, 2006; (5) 34_combinations_final.txt, 96.024 kb, created June 8, 2006; and (6) 00014onco.seq.txt, 4148 kb, created June 12, 2007.

### TECHNICAL FIELD OF THE INVENTION

This invention is related to the area of cancer diagnostics and therapeutics. In particular, it relates to aberrant methylation patterns of particular genes in colon cancer and pre-cancer.

### BACKGROUND OF THE INVENTION

### DNA METHYLATION AND ITS ROLE IN CARCINOGENESIS

The information to make the cells of all living organisms is contained in their DNA. DNA is made up of a unique sequence of four bases: adenine (A), guanine (G), thymine (T) and cytosine (C). These bases are paired A to T and G to C on the two strands that form the DNA double helix. Strands of these pairs store information to make specific molecules grouped into regions called genes. Within each cell, there are processes that control what gene is turned on, or expressed, thus defining the unique function of the cell. One of these control mechanisms is provided by adding a methyl group onto cytosine (C). The methyl group tagged C can be written as mC.

DNA methylation plays an important role in determining whether some genes are expressed or not. By turning genes off that are not needed, DNA methylation is an essential control mechanism for the normal development and functioning of organisms. Alternatively, abnormal DNA methylation is one of the mechanisms underlying the changes observed with aging and development of many cancers.

Cancers have historically been linked to genetic changes caused by chromosomal mutations within the DNA. Mutations, hereditary or acquired, can lead to the loss of expression of genes critical for maintaining a healthy state. Evidence now supports that a relatively large number of cancers are caused by inappropriate DNA methylation, frequently near DNA mutations. In many cases, hyper-methylation of DNA incorrectly switches off critical genes, such as tumor suppressor genes or DNA repair genes, allowing cancers to develop and progress. This non-mutational process for controlling gene expression is described as epigenetics.

DNA methylation is a chemical modification of DNA performed by enzymes called methyltransferases, in which a methyl group (m) is added to certain cytosines (C) of DNA. This non-mutational (epigenetic) process (mC) is a critical factor in gene expression regulation. See, J.G. Herman, Seminars in Cancer Biology, 9: 359-67, 1999.

Although the phenomenon of gene methylation has attracted the attention of cancer researchers for some time, its true role in the progression of human cancers is just now being recognized. In normal cells, methylation occurs predominantly in regions of DNA that have few CG base repeats, while CpG islands, regions of DNA that have long repeats of CG bases, remain non-methylated. Gene promoter regions that control protein expression are often CpG island-rich. Aberrant methylation of these normally non-methylated CpG islands in the promoter region causes transcriptional inactivation or silencing of certain tumor suppressor expression in human cancers.

Genes that are hypermethylated in tumor cells are strongly specific to the tissue of origin of the tumor. Molecular signatures of cancers of all types can be used to improve cancer detection, the assessment of cancer risk and response to therapy. Promoter hypermethylation events provide some of the most promising markers for such purposes.

### PROMOTER GENE HYPERMETHYLATION: PROMISING TUMOR MARKERS

Information regarding the hypermethylation of specific promote genes can be beneficial to diagnosis, prognosis, and treatment of various cancers. Methylation of specific gene promoter regions can occur early and often in carcinogenesis making these markers ideal targets for cancer diagnostics.

Methylation patterns are tumor specific. Positive signals are always found in the same location of a gene. Real time PCR-based methods are highly sensitive, quantitative, and suitable for clinical use. DNA is stable and is found intact in readily available fluids (*e.g.*, serum, sputum, stool, blood, and urine) and paraffin embedded tissues. Panels of pertinent gene markers may cover most human cancers.

### DIAGNOSIS

Key to improving the clinical outcome in patients with cancer is diagnosis at its earliest stage, while it is still localized and readily treatable. The characteristics noted above provide the means for a more accurate screening and surveillance program by identifying higher-risk patients on a molecular basis. It could also provide justification for more definitive follow up of patients who have molecular but not yet all the pathological or clinical features associated with malignancy.

At present, early detection of colorectal cancer is carried out by (1) the "fecal occult blood test" (FOBT), which has a very low sensitivity and specificity, (2) by sigmoidoscopy and/or colonoscopy which is invasive and expensive (and limited in supply), (3) by X-ray detection after double-contrast barium enema, which allows only for the detection of rather large polyps, or CT-colonography (also called virtual colonoscopy) which is still experimental, and (4) by a gene mutation analysis test called PreGen-Plus (Exact Sciences; LabCorp) which is costly and of limited sensitivity.

### PREDICTING TREATMENT RESPONSE

Information about how a cancer develops through molecular events could allow a clinician to predict more accurately how such a cancer is likely to respond to specific chemotherapeutic agents. In this way, a regimen based on knowledge of the tumor's chemosensitivity could be rationally designed. Studies have shown that hypermethylation of the MGMT promoter in glioma patients is indicative of a good response to therapy, greater overall survival and a longer time to progression.

There is a continuing need in the art for new diagnostic and prognostic markers and therapeutic targets for cancer to improve management of patient care.

### SUMMARY OF THE INVENTION

According to one embodiment of the invention, a method is provided for identifying colorectal cancer or predisposition to colorectal cancer. Epigenetic silencing of at least one gene listed in Table 1 is detected in a test sample. The test ample contains colorectal cells or nucleic acids from colorectal cells. The test sample is identified as neoplastic or predisposed to neoplasia.

Another embodiment of the invention is a method of reducing or inhibiting neoplastic growth of a cell which exhibits epigenetic silenced transcription of at least one gene associated with a cancer. An epigenetically silenced gene is determined in a cell. The epigenetically silenced gene is selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899. Expression of a polypeptide encoded by the epigenetic silenced gene is restored in the cell by contacting the cell with a CpG dinucleotide demethylating agent, thereby reducing or inhibiting unregulated growth of the cell.

Another embodiment of the invention is a method of reducing or inhibiting neoplastic growth of a cell which exhibits epigenetic silenced transcription of at least one gene associated with a cancer. An epigenetically silenced gene is determined in a cell. The gene is selected from the group consisting of NM_145059.1, NM_183244.1, NM_145341.2, NM_080672.3, NM_002630, NM_003383, NM_005504, M_016269, NM_006988, NM_021637, NM_001888, NM-014899, NM-145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899. A polynucleotide encoding a polypeptide is introduced into the cell. The polypeptide is encoded by said gene. The polypeptide is expressed in the cell thereby restoring expression of the polypeptide in the cell.

According to yet another aspect of the invention a method of treating a cancer patient is provided. A cancer cell in the patient is determined to have an epigenetic silenced gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899. A demethylating agent is administered to the patient in sufficient amounts to restore expression of the epigenetic silenced gene in the patient's cancer cells.

Still another embodiment of the invention is another method of treating a cancer patient. A cancer cell in the patient is determined to have an epigenetic silenced gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899. A polynucleotide encoding a polypeptide is administered to the patient. The polypeptide is encoded by the epigenetic silenced gene. The polypeptide is expressed in the patient's tumor, thereby restoring expression of the polypeptide in the cancer.

The invention also provides a method for selecting a therapeutic strategy for treating a cancer patient. A gene whose expression in cancer cells of the patient is reactivated by a demethylating agent is identified. The gene is selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, M_015111, NM_002181, NM_005637, NM_030790, NM_018144, NM_004232, NM_030912, NM_145059, NM_033338, NM_006834, NM_003014, NM_001343, NM_000963, NM_004385, and NM_002899. A therapeutic agent which increases expression of the gene is selected for treating said cancer patient.

The present invention also provides a kit for assessing methylation in a cell sample. The kit provides in a package: (1) a reagent that (a) modifies methylated cytosine residues but not non-methylated cytosine residues, or that (b); modifies non-methylated cytosine residues but not methylated cytosine residues; and (2) a pair of oligonucleotide primers that specifically hybridizes under amplification conditions to a region of a gene selected from the group consisting of NM_145059.1, NM_183244.1, NM_145341.2, NM_080672.3, NM_002630, NM_003383, NM 005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899. The region of the gene is within about 1kb of said gene's transcription start site.

These and other embodiments which will be apparent to those of skill in the art upon reading the specification provide the art with tools and methods for detection, diagnosis, prognosis, therapy, and drug selection pertaining to neoplastic cells and cancers.

### BRIEF DESCRIPTION OF THE DRAWINGS AND TABLES

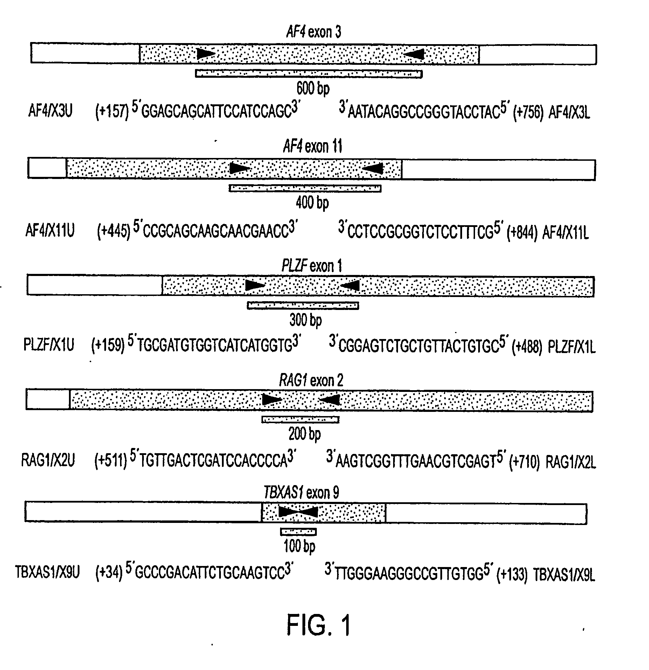

Fig 1. Amplification of genes for quality control of integrity of DNA with primers as follows: AF4 exon 3 (primers SEQ ID NO: 794,795); AF4 exon 11 (primers SEQ ID NO: 796, 797); PLZF exon 1 (primers SEQ ID NO: 798, 799); RAG1 exon 2 (primers SEQ ID NO: 800, 801); and TBXAS1 exon 9 (primes SEQ ID NO: 802, 803). See Example 2.

Table 1. Methylation markers for early detection and prognosis of colon cancer or pre-cancer.

Table 2 is contained on the compact disk which is incorporated herein. Table 2 shows the nucleotide sequences of the methylation markers.

Table 3 is contained on the compact disk which is incorporated herein. Table 3 shows the amino acid sequences of the methylation markers.

Table 4 is contained on the compact disk which is incorporated herein. Table 4 shows the promoter regions of the methylation markers.

Table 5 is contained on the compact disk which is incorporated herein. Table 5 shows combination of markers that can be used.

Table 6 is contained on the compact disk which is incorporated herein. Table 6 shows the splice variants of the markers.

### DETAILED DESCRIPTION OF THE INVENTION

The inventors have discovered a set of genes whose transcription is epigenetically silenced in cancers and pre-cancers. All of the identified genes are shown in Table 1.

Epigenetic silencing of a gene can be determined by any method known in the art. One method is to determine that a gene which is expressed in normal cells is less expressed or not expressed in tumor cells. This method does not, on its own, however, indicate that the silencing is epigenetic, as the mechanism of the silencing could be genetic, for example, by somatic mutation. One method to determine that the silencing is epigenetic is to treat with a reagent, such as DAC (5'-deazacytidine), or with a reagent which changes the histone acetylation status of cellular DNA or any other treatment affecting epigenetic mechanisms present in cells, and observe that the silencing is reversed, *i.e.,* that the expression of the gene is reactivated or restored. Another means to determine epigenetic silencing is to determine the presence of methylated CpG dinucleotide motifs in the silenced gene. Typically these reside near the transcription start site, for example, within about 1 kbp, within about 750 bp, or within about 500 bp. After a gene has been identified as the target of epigenetic silencing in tumor cells, reduced expression of the gene can be used as an indicator of epigenetic silencing.

Expression of a gene can be assessed using any means known in the art. Either mRNA or protein can be measured. Methods employing hybridization to nucleic acid probes can be employed for measuring specific mRNAs. Such methods include using nucleic acid probe arrays (microarray technology), in situ hybridization, and using Northern blots. Messenger RNA can also be assessed using amplification techniques, such as RT-PCR. Advances in genomic technologies now permit the simultaneous analysis of thousands of genes, although many are based on the same concept of specific probe-target hybridization. Sequencing-based methods are an alternative; these methods started with the use of expressed sequence tags (ESTs), and now include methods based on short tags, such as serial analysis of gene expression (SAGE) and massively parallel signature sequencing (MPSS). Differential display techniques provide yet another means of analyzing gene expression; this family of techniques is based on random amplification of cDNA fragments generated by restriction digestion, and bands that differ between two tissues identify cDNAs of interest. Specific proteins can be assessed using any convenient method including immunoassays and immuno-cytochemistry but are not limited to that. Most such methods will employ antibodies which are specific for the particular protein or protein fragments. The sequences of the mRNA (cDNA) and proteins of the markers of the present invention are provided in the sequence listing.

Methylation-sensitive restriction endonucleases can be used to detect methylated CpG dinucleotide motifs. Such endonucleases may either preferentially cleave methylated recognition sites relative to non-methylated recognition sites or preferentially cleave non-methylated relative to methylated recognition sites. Examples of the former are Acc III, Ban I, BstN I, Msp I, and Xma I. Examples of the latter are Acc II, Ava I, BssH II, BstU I, Hpa II, and Not I. Alternatively, chemical reagents can be used which selectively modify either the methylated or non-methylated form of CpG dinucleotide motifs.

Modified products can be detected directly, or after a further reaction which creates products which are easily distinguishable. Means which detect altered size and/or charge can be used to detect modified products, including but not limited to electrophoresis, chromatography, and mass spectrometry. Examples of such chemical reagents for selective modification include hydrazine and bisulfite ions. Hydrazine-modified DNA can be treated with piperidine to cleave it. Bisulfite ion-treated DNA can be treated with alkali.

A variety of amplification techniques may be used in a reaction for creating distinguishable products. Some of these techniques employ PCR. Other suitable amplification methods include the ligase chain reaction (LCR) (Barringer et al, 1990), transcription amplification (Kwoh et al. 1989; WO88/10315), selective amplification of target polynucleotide sequences (US Patent No. 6,410,276), consensus sequence primed polymerase chain reaction (US Patent No 4,437,975), arbitrarily primed polymerase chain reaction (WO90/06995), nucleic acid based sequence amplification (NASBA) (US Patent Nos 5,409,818; 5,554,517; 6,063,603), nick displacement amplification (WO2004/067726).

Sequence variation that reflects the methylation status at CpG dinucleotides in the original genomic DNA offers two approaches to PCR primer design. In the first approach, the primers do not themselves "cover" or hybridize to any potential sites of DNA methylation; sequence variation at sites of differential methylation are located between the two primers. Such primers are used in bisulphite genomic sequencing, COBRA, Ms-SNuPE. In the second approach, the primers are designed to anneal specifically with either the methylated or unmethylated version of the converted sequence. If there is a sufficient region of complementarity, e.g., 12, 15, 18, or 20 nucleotides, to the target, then the primer may also contain additional nucleotide residues that do not interfere with hybridization but may be useful for other manipulations. Exemplary of such other residues may be sites for restriction endonuclease cleavage, for ligand binding or for factor binding or linkers or repeats. The oligonucleotide primers may or may not be such that they are specific for modified methylated residues

One way to distinguish between modified and unmodified DNA is to hybridize oligonucleotide primers which specifically bind to one form or the other of the DNA. After hybridization, an amplification reaction can be performed and amplification products assayed. The presence of an amplification product indicates that a sample hybridized to the primer. The specificity of the primer indicates whether the DNA had been modified or not, which in turn indicates whether the DNA had been methylated or not. For example, bisulfite ions modify non-methylated cytosine bases, changing them to uracil bases. Uracil bases hybridize to adenine bases under hybridization conditions. Thus an oligonucleotide primer which comprises adenine bases in place of guanine bases would hybridize to the bisulfite-modified DNA, whereas an oligonucleotide primer containing the guanine bases would hybridize to the non-modified (methylated) cytosine residues in the DNA. Amplification using a DNA polymerase and a second primer yield amplification products which can be readily observed. Such a method is termed MSP (Methylation Specific PCR; Patent Nos 5,786,146; 6,017,704; 6,200,756). The amplification products can be optionally hybridized to specific oligonucleotide probes which may also be specific for certain products. Alternatively, oligonucleotide probes can be used which will hybridize to amplification products from both modified and nonmodified DNA.

Another way to distinguish between modified and nonmodified DNA is to use oligonucleotide probes which may also be specific for certain products. Such probes can be hybridized directly to modified DNA or to amplification products of modified DNA. Oligonucleotide probes can bye labeled using any detection system known in the art. These include but are not limited to fluorescent moieties, radioisotope labeled moieties, bioluminescent moieties, luminescent moieties, chemiluminescent moieties, enzymes, substrates, receptors, or ligands.

Still another way for the identification of methylated CpG dinucleotides utilizes the ability of the MBD domain of the McCP2 protein to selectively bind to methylated DNA sequences (Cross et al, 1994; Shiraishi et al, 1999). Restriction enconuclease digested genomic DNA is loaded onto expressed His-tagged methyl-CpG binding domain that is immobilized to a solid matrix and used for preparative column chromatography to isolate highly methylated DNA sequences.

Real time chemistry allow for the detection of PCR amplification during the early phases of the reactions, and makes quantitation of DNA and RNA easier and more precise. A few variations of the real-time PCR are known. They include the TaqMan system and Molecular Beacon system which have separate probes labeled with a fluorophore and a fuorescence quencher. In the Scorpion system the labeled probe in the form of a hairpin structure is linked to the primer. ,

DNA methylation analysis has been performed successfully with a number of techniques which include the MALDI-TOFF, MassARRAY, MethyLight, Quantitative analysis of ethylated alleles (QAMA), enzymatic regional methylation assay (ERMA), HeavyMethyl, QBSUPT, MS-SNuPE, MethylQuant, Quantitative PCR sequencing, and Oligonucleotide-based microarray systems.

The number of genes whose silencing is tested and/or detected can vary: one, two, three, four, five, or more genes can be tested and/or detected. In some cases at least two genes are selected. In other embodiments at least three genes are selected. Exemplary combinations useful for testing and determining are shown in Table 5.

Testing can be performed diagnostically or in conjunction with a therapeutic regimen. Testing can be used to monitor efficacy of a therapeutic regimen, whether a chemotherapeutic agent or a biological agent, such as a polynucleotide. Testing can also be used to determine what therapeutic or preventive regimen to employ on a patient. Moreover, testing can be used to stratify patients into groups for testing agents and determining their efficacy on various groups of patients. The test of the present invention can be used in conjunction with other factors to provide a diagnosis. A skilled practitioner in the art knows that many factors are considered and weighed in making a diagnosis. These may include other genetic markers, as well as physical findings, radiological findings, histology, etc. Thus the markers of the invention may be used to aid in formulating a diagnosis. Moreover, a lab test can be performed and evaluated and a determination with respect to a marker recorded in a tangible medium, such as a paper or electronic record. Providing a diagnosis to a patient or other physician can be done by a physician in writing, orally, or electronically.

Test samples for diagnostic, prognostic, or personalized medicine uses can be obtained from surgical samples, such as biopsies or fine needle aspirates, from paraffin embedded colon, recturn, small intestinal, gastric, esophageal, bone marrow, breast, ovary, prostate, kidney, lung, brain on other organ tissues, from a body fluid such as blood, serum, lymph, cerebrospinal fluid, saliva, sputum, bronchial -lavage fluid, ductal fluids stool, urine, lymph nodes, or semen. Such sources are not meant to be exhaustive, but rather exemplary. A test sample obtainable from such specimens or fluids includes detached tumor cells or free nucleic acids that are released from dead or damaged tumor cells. Nucleic acids include RNA, genomic DNA, mitochondrial DNA, single or double stranded, and protein-associated nucleic acids. Any nucleic acid specimen in purified or non-purified form obtained from such specimen cell can be utilized as the starting nucleic acid or acids.

Demethylating agents can be contacted with cells *in vitro* or *in vivo* for the purpose of restoring normal gene expression to the cell. Suitable demethylating agents include, but are not limited to 5-aza-2'-deoxycytidine, 5-aza-cytidine, Zebularine, procaine, and L-ethionine. This reaction may be used for diagnosis, for determining predisposition, and for determining suitable therapeutic regimes. If the demethylating agent is used for treating colon, head and neck, esophageal, gastric, pancreatic, or liver cancers, expression or methylation can be tested of a gene selected from the group shown in Table 1.

An alternative way to restore epigenetically silenced gene expression is to introduce a non-methylated polynucleotide into a cell, so that it will be expressed in the cell. Various gene therapy vectors and vehicles are known in the art and any can be used as is suitable for a particular situation. Certain vectors are suitable for short term expression and certain vectors are suitable for prolonged expression. Certain vectors are trophic for certain organs and these can be used as is appropriate in the particular situation. Vectors may be viral or non-viral. The polynucleotide can, but need not, be contained in a vector, for example, a viral vector, and can be formulated, for example, in a matrix such as a liposome, microbubbles. The polynucleotide can be introduced into a cell by administering the polynucleotide to the subject such that it contacts the cell, is taken up by the cell, and the encoded polypeptide is expressed. Suitable polynucleotides are provided in the sequence listing as SEQ ID NO: 273 to 532. Polynucleotides and/or oligonucleotides encoding the polypeptides shown in SEQ ID NO: 533-793 can also be used. Preferably the specific polynucleotide will be one which the patient has been tested for and been found to carry a silenced version. The polynucleotides for treating colon, head and neck, esophageal, gastric, pancreas, liver cancers will typically encode a gene selected from those shown in Table 1.

Cells exhibiting methylation silenced gene expression generally are contacted with the demethylating agent in vivo by administering the agent to a subject. Where convenient, the demethylating agent can be administered using, for example, a catheterization procedure, at or near the site of the cells exhibiting unregulated growth in the subject, or into a blood vessel in which the blood is flowing to the site of the cells. Similarly, where an organ, or portion thereof, to be treated can be isolated by a shunt procedure, the agent can be administered via the shunt, thus substantially providing the agent to the site containing the cells. The agent also can be administered systemically or via other routes known in the art.

The polynucleotide can include, in addition to polypeptide coding sequence, operatively linked transcriptional regulatory elements, translational regulatory elements, and the like, and can be in the form of a naked DNA molecule, which can be contained in a vector, or can be formulated in a matrix such as a liposome or microbubbles that facilitates entry of the polynucleotide into the particular cell. The term "operatively linked" refers to two or more molecules that are positioned with respect to each other such that they act as a single unit and effect a function attributable to one or both molecules or a combination thereof. A polynucleotide sequence encoding a desired polypeptide can be operatively linked to a regulatory element, in which case the regulatory element confers its regulatory effect on the polynucleotide similar to the way in which the regulatory element would affect a polynucleotide sequence with which it normally is associated with in a cell.

The polynucleotide encoding the desired polypeptide to be administered to a mammal or a human or to be contacted with a cell may contain a promoter sequence, which can provide constitutive or, if desired, inducible or tissue specific or developmental stage specific expression of the polynucleotide, a polyA recognition sequence, and a ribosome recognition site or internal ribosome entry site, or other regulatory elements such as an enhancer, which can be tissue specific. The vector also may contain elements required for replication in a prokaryotic or eukaryotic host system or both, as desired. Such vectors, which include plasmid vectors and viral vectors such as bacteriophage, baculovirus, retrovirus, lentivirus, adenovirus, vaccinia virus, semliki forest virus and adeno-associated virus vectors, are well known and can be purchased from a commercial source (Promega, Madison WI.; Stratagene, La Jolla CA.; GIBCO/BRL, Gaithersburg MD.) or can be constructed by one skilled in the art (see, for example, Meth. Enzymol., Vol. 185, Goeddel, ed. (Academic Press, Inc., 1990); Jolly, Canc. Gene Ther. 1:51-64, 1994; Flotte, J. Bioenerg. Biomemb. 25:37-42, 1993; Kirshenbaum et al., J. Clin. Invest. 92:381-387, 1993; each of which is incorporated herein by reference).

A tetracycline (tet) inducible promoter can be used for driving expression of a polynucleotide encoding a desired polypeptide. Upon administration of tetracycline, or a tetracycline analog, to a subject containing a polynucleotide operatively linked to a tet inducible promoter, expression of the encoded polypeptide is induced. The polynucleotide alternatively can be operatively linked to tissue specific regulatory element, for example, a liver cell specific regulatory element such as an α.-fetoprotein promoter (Kanai et al., Cancer Res. 57:461-465, 1997; He et al., J. Exp. Clin. Cancer Res. 19:183-187, 2000) or an albumin promoter (Power et al., Biochem. Biophys. Res. Comm. 203:1447-1456, 1994; Kuriyama et al., Int. J. Cancer 71:470-475, 1997); a muscle cell specific regulatory element such as a myoglobin promoter (Devlin et al., J. Biol. Chem. 264:13896-13901, 1989; Yan et al., J. Biol. Chem. 276:17361-17366, 2001); a prostate cell specific regulatory element such as the PSA promoter (Schuur et al., J. Biol. Chem. 271:7043-7051, 1996; Latham et al., Cancer Res. 60:334-341, 2000); a pancreatic cell specific regulatory element such as the elastase promoter (Ornitz et al., Nature 313:600-602, 1985; Swift et al., Genes Devel. 3:687-696, 1989); a leukocyte specific regulatory element such as the leukosialin (CD43) promoter (Shelley et al., Biochem. J. 270:569-576, 1990; Kudo and Fukuda, J. Biol. Chem. 270:13298-13302, 1995); or the like, such that expression of the polypeptide is restricted to particular cell in an individual, or to particular cells in a mixed population of cells in culture, for example, an organ culture. Regulatory elements, including tissue specific regulatory elements, many of which are commercially available, are well known in the art (see, for example, InvivoGen; San Diego Calif.).

Viral expression vectors can be used for introducing a polynucleotide into a cell, particularly a cell in a subject. Viral vectors provide the advantage that they can infect host cells with relatively high efficiency and can infect specific cell types. For example, a polynucleotide encoding a desired polypeptide can be cloned into a baculovirus vector, which then can be used to infect an insect host cell, thereby providing a means to produce large amounts of the encoded polypeptide. Viral vectors have been developed for use in particular host systems, particularly mammalian systems and include, for example, retroviral vectors, other lentivirus vectors such as those based on the human immunodeficiency virus (HIV), adenovirus vectors, adeno-associated virus vectors, herpesvirus vectors, hepatitis virus vectors, vaccinia virus vectors, and the like (see Miller and Rosman, BioTechniques 7:980-990, 1992; Anderson et al., Nature 392:25-30 Suppl., 1998; Verma and Somia, Nature 389:239-242, 1997; Wilson, New Engl. J. Med. 334:1185-1187 (1996), each of which is incorporated herein by reference).

A polynucleotide, which can optionally be contained in a vector, can be introduced into a cell by any of a variety of methods known in the art (Sambrook et al., supra, 1989; Ausubel et al., Current Protocols in Molecular Biology, John Wiley and Sons, Baltimore, Md. (1987, and supplements through 1995), each of which is incorporated herein by reference). Such methods include, for example, transfection, lipofection, microinjection, electroporation and, with viral vectors, infection; and can include the use of liposomes, microemulsions or the like, which can facilitate introduction of the polynucleotide into the cell and can protect the polynucleotide from degradation prior to its introduction into the cell. A particularly useful method comprises incorporating the polynucleotide into microbubbles, which can be injected into the circulation. An ultrasound source can be positioned such that ultrasound is transmitted to the tumor, wherein circulating microbubbles containing the polynucleotide are disrupted at the site of the tumor due to the ultrasound, thus providing the polynucleotide at the site of the cancer. The selection of a particular method will depend, for example, on the cell into which the polynucleotide is to be introduced, as well as whether the cell is in culture or *in situ* in a body.

Introduction of a polynucleotide into a cell by infection with a viral vector can efficiently introduce the nucleic acid molecule into a cell. Moreover, viruses are very specialized and can be selected as vectors based on an ability to infect and propagate in one or a few specific cell types. Thus, their natural specificity can be used to target the nucleic acid molecule contained in the vector to specific cell types. A vector based on an HIV can be used to infect T cells, a vector based on an adenovirus can be used, for example, to infect respiratory epithelial cells, a vector based on a herpesvirus can be used to infect neuronal cells, and the like. Other vectors, such as adeno-associated viruses can have greater host cell range and, therefore, can be used to infect various cell types, although viral or non-viral vectors also can be modified with specific receptors or ligands to alter target specificity through receptor mediated events. A polynucleotide of the invention, or a vector containing the polynucleotide can be contained in a cell, for example, a host cell, which allows propagation of a vector containing the polynucleotide, or a helper cell, which allows packaging of a viral vector containing the polynucleotide. The polynucleotide can be transiently contained in the cell, or can be stably maintained due, for example, to integration into the cell genome.

A polypeptide according to any of SEQ ID NO 533-793 can be administered directly to the site of a cell exhibiting unregulated growth in the subject. The polypeptide can be produced and isolated, and formulated as desired, using methods as disclosed herein, and can be contacted with the cell such that the polypeptide can cross the cell membrane of the target cells. The polypeptide may be provided as part of a fusion protein, which includes a peptide or polypeptide component that facilitates transport across cell membranes. For example, a human immunodeficiency virus (HIV) TAT protein transduction domain or a nuclear localization domain may be fused to the marker of interest. The administered polypeptide can be formulated in a matrix that facilitates entry of the polypeptide into a cell.

While particular polynucleotide and polypeptide sequences are mentioned here as representative of known genes and proteins, those of skill in the art will understand that the sequences in the databases represent the sequences present in particular individuals. Any allelic sequences from other individuals can be used as well. These typically vary from the disclosed sequences at 1-10 residues, at 1-5 residues, or at 1-3 residues. Moreover, the allelic sequences are typically at least 95, 96, 97, 98, or 99 % identical to the database sequence, as measured using an algorithm such as the BLAST homology tools.

An agent such as a demethylating agent, a polynucleotide, or a polypeptide is typically formulated in a composition suitable for administration to the subject. Thus, the invention provides compositions containing an agent that is useful for restoring regulated growth to a cell exhibiting unregulated growth due to methylation silenced transcription of one or more genes. The agents are useful as medicaments for treating a subject suffering from a pathological condition associated with such unregulated growth. Such medicaments generally include a carrier. Acceptable carriers are well known in the art and include, for example, aqueous solutions such as water or physiologically buffered saline or other solvents or vehicles such as glycols, glycerol, oils such as olive oil or injectable organic esters. An acceptable carrier can contain physiologically acceptable compounds that act, for example, to stabilize or to increase the absorption of the conjugate. Such physiologically acceptable compounds include, for example, carbohydrates, such as glucose, sucrose or dextrans, antioxidants, such as ascorbic acid or glutathione, chelating agents, low molecular weight proteins or other stabilizers or excipients. One skilled in the art would know or readily be able to determine an acceptable carrier, including a physiologically acceptable compound. The nature of the carrier depends on the physico-chemical characteristics of the therapeutic agent and on the route of administration of the composition. Administration of therapeutic agents or medicaments can be by the oral route or parenterally such as intravenously, intramuscularly, subcutaneously, transdermally, intranasally, intrabronchially, vaginally, rectally, intratumorally, or other such method known in the art. The pharmaceutical composition also can contain one more additional therapeutic agents.

The therapeutic agents can be incorporated within an encapsulating material such as into an oil-in-water emulsion, a microemulsion, micelle, mixed micelle, liposome, microsphere, microbubbles or other polymer matrix (see, for example, Gregoriadis, Liposome Technology, Vol. 1 (CRC Press, Boca Raton, Fla. 1984); Fraley, et al., Trends Biochem. Sci., 6:77 (1981), each of which is incorporated herein by reference). Liposomes, for example, which consist of phospholipids or other lipids, are nontoxic, physiologically acceptable and metabolizable carriers that are relatively simple to make and administer. "Stealth" liposomes (see, for example, U.S. Pat. Nos. 5,882,679; 5,395,619; and 5,225,212, each of which is incorporated herein by reference) are an example of such encapsulating materials particularly useful for preparing a composition useful in a method of the invention, and other "masked" liposomes similarly can be used, such liposomes extending the time that the therapeutic agent remain in the circulation. Cationic liposomes, for example, also can be modified with specific receptors or ligands (Morishita et al., J. Clin. Invest., 91:2580-2585 (1993), which is incorporated herein by reference). In addition, a polynucleotide agent can be introduced into a cell using, for example, adenovirus-polylysine DNA complexes (see, for example, Michael et al., J. Biol. Chem. 268:6866-6869 (1993), which is incorporated herein by reference).

The route of administration of the composition containing the therapeutic agent will depend, in part, on the chemical structure of the molecule. Polypeptides and polynucleotides, for example, are not efficiently delivered orally because they can be degraded in the digestive tract. However, methods for chemically modifying polypeptides, for example, to render them less susceptible to degradation by endogenous proteases or more absorbable through the alimentary tract may be used (see, for example, Blondelle et al., supra, 1995; Ecker and Crook, supra, 1995).

The total amount of an agent to be administered in practicing a method of the invention can be administered to a subject as a single dose, either as a bolus or by infusion over a relatively short period of time, or can be administered using a fractionated treatment protocol, in which multiple doses are administered over a prolonged period of time. One skilled in the art would know that the amount of the composition to treat a pathologic condition in a subject depends on many factors including the age and general health of the subject as well as the route of administration and the number of treatments to be administered. In view of these factors, the skilled artisan would adjust the particular dose as necessary. In general, the formulation of the composition and the routes and frequency of administration are determined, initially, using Phase I and Phase II clinical trials.

The composition can be formulated for oral formulation, such as a tablet, or a solution or suspension form; or can comprise an admixture with an organic or inorganic carrier or excipient suitable for enteral or parenteral applications, and can be compounded, for example, with the usual non-toxic, pharmaceutically acceptable carriers for tablets, pellets, capsules, suppositories, solutions, emulsions, suspensions, or other form suitable for use. The carriers, in addition to those disclosed above, can include glucose, lactose, mannose, gum acacia, gelatin, mannitol, starch paste, magnesium trisilicate, talc, corn starch, keratin, colloidal silica, potato starch, urea, medium chain length triglycerides, dextrans, and other carriers suitable for use in manufacturing preparations, in solid, semisolid, or liquid form. In addition auxiliary, stabilizing, thickening or coloring agents and perfumes can be used, for example a stabilizing dry agent such as triulose (see, for example, U.S. Pat. No. 5,314,695).

Although diagnostic and prognostic accuracy and sensitivity may be achieved by using a combination of markers, such as 5 or 6 markers, or 9 or 10 markers, or 14 or 15 markers, practical considerations may dictate use of smaller combinations. Any combination of markers for a specific cancer may be used which comprises 2, 3, 4, or 5 markers. Combinations of 2, 3, 4, or 5 markers can be readily envisioned given the specific disclosures of individual markers provided herein. Exemplary combinations useful for testing and determining are shown in Table 5.

The level of methylation of the differentially methylated GpG islands can provide a variety of information about the disease or cancer. It can be used to diagnose a disease or cancer in the individual. Alternatively, it can be used to predict the course of the disease or cancer in the individual or to predict the suspectibility to disease or cancer or to stage the progression of the disease or cancer in the individual. It can help to predict the likelihood of overall survival or predict the likelihood of reoccurrence of disease or cancer and to determine the effectiveness of a treatment course undergone by the individual. Increase or decrease of methylation levels in comparison with reference level and alterations in the increase/decrease when detected provide useful prognostic and diagnostic value.

The prognostic methods can be used to identify patients with adenomas that are likely to progress to carcinomas. Such a prediction can be made on the basis of epigenetic silencing of one of the genes identified in Table 1 in an adenoma relative to normal tissue. Such patients can be offered additional appropriate therapeutic or preventative options, including endoscopic polypectomy or resection, and when indicated, surgical procedures, chemotherapy, radiation, biological response modifiers, or other therapies. Such patients may also receive recommendations for further diagnostic or monitoring procedures, including but not limited to increased frequency of colonoscopy, virtual colonoscopy, video capsule endoscopy, PET-CT, molecular imaging, or other imaging techniques.

A therapeutic strategy for treating a cancer patient can be selected based on reactivation of epigenetically silenced genes. First a gene selected from those listed in Table 1 is identified whose expression in cancer cells of the patient is reactivated by a demethylating agent or epigenetically silenced. A treatment which increases the expression of the gene is then selected. Such a treatment can comprise administration of a reactivating agent or a polynucleotide. A polypeptide can alternatively be administered.

Kits according to the present invention are assemblages of reagents for testing methylation. They are typically in a package which contains all elements, optionally including instructions. The package may be divided so that components are not mixed until desired. Components may be in different physical states. For example, some components may be lyophilized and some in aqueous solution. Some may be frozen. Individual components may be separately packaged within the kit. The kit may contain reagents, as described above for differentially modifying methylated and non-methylated cytosine residues. Desirably the kit will contain oligonucleotide primers which specifically hybridize to regions within 1 kb of the transcription start sites of the genes/markers identified in the attached Table 1. Typically the kit will contain both a forward and a reverse primer for a single gene or marker. If there is a sufficient region of complementarity, e.g., 12, 15, 18, or 20 nucleotides, then the primer may also contain additional nucleotide residues that do not interfere with hybridization but may be useful for other manipulations. Exemplary of such other residues may be sites for restriction endonuclease cleavage, for ligand binding or for factor binding or linkers or repeats. The oligonucleotide primers may or may not be such that they are specific for modified methylated residues. The kit may optionally contain oligonucleotide probes. The probes may be specific for sequences containing modified methylated residues or for sequences containing non-methylated residues. The kit may optionally contain reagents for modifying methylated cytosine residues. The kit may also contain components for performing amplification, such as a DNA polymerase and deoxyribonucleotides. Means of detection may also be provided in the kit, including detectable labels on primers or probes. Kits may also contain reagents for detecting gene expression for one of the markers of the present invention (Table 1). Such reagents may include probes, primers, or antibodies, for example. In the case of enzymes or ligands, substrates or binding partners may be sued to assess the presence of the marker.

In one aspect of this embodiment, the gene is contacted with hydrazine, which modifies cytosine residues, but not methylated cytosine residues, then the hydrazine treated gene sequence is contacted with a reagent such as piperidine, which cleaves the nucleic acid molecule at hydrazine modified cytosine residues, thereby generating a product comprising fragments. By separating the fragments according to molecular weight, using, for example, an electrophoretic, chromatographic, or mass spectrographic method, and comparing the separation pattern with that of a similarly treated corresponding non-methylated gene sequence, gaps are apparent at positions in the test gene contained methylated cytosine residues. As such, the presence of gaps is indicative of methylation of a cytosine residue in the CpG dinucleotide in the target gene of the test cell.

Bisulfite ions, for example, sodium bisulfite, convert non-methylated cytosine residues to bisulfite modified cytosine residues. The bisulfite ion treated gene sequence can be exposed to alkaline conditions, which convert bisulfite modified cytosine residues to uracil residues. Sodium bisulfite reacts readily with the 5,6-double bond of cytosine (but poorly with methylated cytosine) to form a sulfonated cytosine reaction intermediate that is susceptible to deamination, giving rise to a sulfonated uracil. The sulfonate group can be removed by exposure to alkaline conditions, resulting in the formation of uracil. The DNA can be amplified, for example, by PCR, and sequenced to determine whether CpG sites are methylated in the DNA of the sample. Uracil is recognized as a thymine by Taq polymerase and, upon PCR, the resultant product contains cytosine only at the position where 5-methylcytosine was present in the starting template DNA. One can compare the amount or distribution of uracil residues in the bisulfite ion treated gene sequence of the test cell with a similarly treated corresponding non-methylated gene sequence. A decrease in the amount or distribution of uracil residues in the gene from the test cell indicates methylation of cytosine residues in CpG dinucleotides in the gene of the test cell. The amount or distribution of uracil residues also can be detected by contacting the bisulfite ion treated target gene sequence, following exposure to alkaline conditions, with an oligonucleotide that selectively hybridizes to a nucleotide sequence of the target gene that either contains uracil residues or that lacks uracil residues, but not both, and detecting selective hybridization (or the absence thereof) of the oligonucleotide.

Test compounds can be tested for their potential to treat cancer. Cancer cells for testing can be selected from the group consisting of prostate, lung, breast, and colon cancer. Expression of a gene selected from those listed in Table 1 is determined and if it is increased by the compound in the cell or if methylation of the gene is decreased by the compound in the cell, one can identify it as having potential as a treatment for cancer.

Alternatively such tests can be used to determine an esophageal, head and neck, gastric, small intestinal, pancreas, liver cancer patient's response to a chemotherapeutic agent. The patient can be treated with a chemotherapeutic agent. If expression of a gene selected from those listed in Table 1 is increased by the compound in cancer cells or if methylation of the gene is decreased by the compound in cancer cells it can be selected as useful for treatment of the patient.

The above disclosure generally describes the present invention. All references disclosed herein are expressly incorporated by reference. A more complete understanding can be obtained by reference to the following specific examples which are provided herein for purposes of illustration only, and are not intended to limit the scope of the invention.

### EXAMPLES

### Example 1- Materials and Methods

**Sample collection and nucleic acids extraction**

Sixty eight colon tumour samples, 37 adenomas and 31 carcinomas, were collected directly after polypectomy or surgery, respectively, evaluated by the pathologist and a tissue sample of these specimens was frozen in liquid nitrogen.

Isolation of RNA and DNA was done with Trizol solution (Invitrogen life technologies), following the supplier instructions. Two 4 µm thick, frozen sections before and after obrtainint the tissue sample for RNA and DNA isolation, respectively, were cut and stained with haematoxylin-eosin in order to estimate the percentage of tumour cells present in the tissue sample. Only samples with at least 70% tumor cells were selected for RNA and DNA isolation.

**Microarray slide preparation**

Oligonucleotide arrays containing 18,861 60mer oligonucleotide sequences representing 18,664 unique human genes, designed by Compugen (San Jose, CA, USA) and obtained from Sigma-Genosys (Zwijndrecht, The Netherlands) were used for microarray expression analysis. The arrays were prepared at the VUmc Microarray facility (on the world-wide web at the domain .vumc.nl at the page microarrays). In short, oligonucleotides were spotted at a concentration of 10 µM, in 150mM sodium phosphate (pH 8.5), on CodeLink slides (Amersham BioSciences, Roosendaal, The Netherlands), using a SpotArray Enterprise (Perkin Elmer Life Sciences, Zaventem, Belgium), and processed according to the manufacturer's protocol.

**cDNA synthesis, probe preparation and hybridisation**

cDNA was prepared from 15 µg of total sample RNA and 15 µg of Universal Human Reference RNA (Stratagene, La Jolla, CA, USA) using oligo-dT₂₀-VN primer (Invitrogen, Breda, The Netherlands), Superscript II Reverse Transcriptase (Invitrogen) and an amino-allyl dNTP stock solution, containing 20mM dATP, 20mM dCTP, 20mM, dGTP, 4mM dTTP (all from Invitrogen) and 16mM amino-allyl-dUTP (Ambion, Cambridgeshire, UK). cDNA was purified using microcon YM-30 filter columns (Millipore B.V., Amsterdam, The Netherlands), completely dried in a vacuum centrifuge and dissolved in 9 µl of 50 mM NaHCO₃/Na₂CO₃ (pH 9.0). Next, cDNA was left to couple to Cy3 (sample) or Cy5 (reference) dyes (Fluorolink Cy3/Cy5 Monofunctional dye pack (Amersham Biosciences)) at room temperature for one hour, after which uncoupled dyes were blocked by adding 4 M hydroxylamine. Cy3 (sample) and Cy5 (reference) labeled cDNA was mixed and the probe was purified using the Qiaquick PCR purification kit (Qiagen Benelux B.V., Venlo, The Netherlands). Twelve µg poly A (Pharmacia, Capelle a/d IJssel, The Netherlands), 60 µg yeast tRNA (Sigma) and 24 µg Cot-1 DNA (Invitrogen) was added after which the probe was precipitated, dissolved in 127 µl hybridisation mix (0.2% SDS, 8% glycerol, 50% formamide and 0.1% dextrane sulphate in 2x SSC), denaturated at 70°C for 10 minutes and incubated at 37°C for one hour. Slides were pre-hybridised for 45 minutes at 37°C with a pre-hybridisation solution containing 30 µg salmon sperm DNA (Gibco, Breda, The Netherlands), 12 µg poly A (Pharmacia), 60 µg yeast tRNA (Sigma) and 24 µg Cot-1 DNA (Invitrogen) dissolved in 127 µl hybridisation mix. Pre-hybridisation was followed by probe hybridisation for 14 hours at 37°C. Both pre-hybridisation and hybridisation were performed in HybStation 12 (Perkin Elmer Life Sciences).

**Data analysis**

*Image aqcuisition and quantification*

Arrays were scanned using ScanArray Express (Perkin Elmer Life Sciences) and quantified in ImaGene 5.6.1 software (BioDiscovery Ltd, Marina del Rey, CA, USA) using default settings for the flagging of bad quality spots. Flagged spots were excluded from further analysis.

*Array analysis*

We analyzed 68 chips containing 30000 genes each. Thirty-seven slides were analyzed using RNA from adenomas and 31 used carcinoma DNA. A GAL file supplied by the VUMC microarray group (galfile="H30K 25042004 b34.gal") was used to correlate spot position and gene name. We used the R based Bioconductor packages (marray) to normalize/analyze the expression data.

We normalized using a median based procedure across arrays ("maNorm"-routine from the "marray" package). The universal standard is always labeled RED [R]. The ratio M is defined as 2log(R/G) (A = 2log(R*G)/2).

We calculated the statistical difference between Adenomas and Carcinomas using the Wilcoxon test on the M ratios. In addition to Wilcoxon we also applied the "Thas" test which is able to detect differential behaviour in (sub)populations.

Progression marker definition: (Median Carcinoma Ratios M) - (Median Adenoma Ratios M) > 0 AND Wilcoxon p-value < 0.00001. This value of 0.00001 is corrected for multiple hypothesis testing.

Early marker definition: (Median Carcinoma Ratios M + Medium Adenoma Ratios M), sort descending (the higher the score the lower the expression), arbitrary cut-off of 4

The promoters of the genes passing the progression or early detection marker definition have to be located on the genome-wide alignment less than five ancestral nodes from an established list of 56 markers (see BROAD promoter analysis) OR have to contain more than or equal to 4 different motifs (see DEEP promoter analysis)

BROAD analysis: Genome-wide promoter Alignment

We extracted 8793 sequences from the Database of Transciption Start Sites (DBTSS, version 3.0 based on human assembly build 31). Subsequently, Newcpgreport was used to detect CpG island in a 500bp region for each gene. A CpG island is defined as a region of minimal 200bp, a GC content larger than 50% and an Observed/Expected (O/E) ratio larger than 0.60. The 500bp are located from -300 to +200 relative to the TSS. We complemented this set with 56 reported/known cancer-specifically methylated genes. This resulted in a sequence set of 4738 genes which are subsequently aligned by clustalw. A software tool called Treeillustrator was used to visualize the large guide tree in addition to indicating the location of the known markers.

*DEEP analysis: Specific Binding Patterns*

The DEEP part of the computational promoter analysis focuses on identifying discrimating sequences feature between two different functional classes (A & B) of CpG island containing promoters. Class A lists genes which are only methylated in cancer and not in normals, while Class B enumerates genes which are at least partially methylated in normals. For each of these genes we extracted a symmetric region of 1kb around the predicted transcription start site (TSS). Again newcpgreport was used and a CpG island is defined as a region of minimal 200bp, a GC content larger than 50% and a O/E ratio larger than 0.60. Seven motifs were found to be overrepresented in Class A versus Class B and were used to predict informative cancer specific methylation.

### EXAMPLE 2

### Material and Methods

Samples arrive as PEFF or fresh frozen material and are subdivided in 3 classes: C = Cancer (region indicated by the pathologist); N = Normal (non cancer material from patient, histologically normal resection end); NN = Normal normal (autopsie material from 'healty' patients).

gDNA is extracted by the classic phenol/chloroform extraction method and measured on the Nanodrop spectrophotometer (Isogen). 1-1.5 µg gDNA is BT treated with the EZ-96 DNA Methylation Kit™ (ZymoResearch) and eluted in 50µl. Integrity test is done on the BT-treated samples with primers to check the length of the DNA fragments. Integrity test primers are shown in Fig. 1 and SEQ ID NO: 794-803. Quality Control is acceptable if we find back amplicons up to 300 bps.

All samples are run on the LightCycler (see LightCycler protocol) with a bACT STD curve and an bACT beacon MB6 (mCGACTGCGTGTGGGGTGGTGATGGAG-GAGGTTTAGGCAGTCGv; SEQ ID NO: 804), bACT copy number is determined. Samples with a copy number < 200 are discarded, samples are normalized to the same copy number.

The following sense (S), antisense (A), and Beacon (B) probes were used for FUK, PDCD4, and PHACTR3:.

| **SYBRGreen primers** | | | | |
|---|---|---|---|---|
| FUK_11670 | S | ATTATATTTTTCGGAGTGTATC GC (SEQ ID NO: 805) | A | TAACGTACCTACCGTCGCCC (SEQ ID NO: 806) |
| PDCD4_11827 | S | GTTCGTAGTTCGGGGCGTT (SEQ ID NO:807 ) | A | GCGATCCTATCAAATCCGAA (SEQ ID NO: 808) |
| PHACTR3_11706 | S | TAGGGAGCGTTAGGTTCGG(S EQ ID NO: 809) | A | CGCTATAAAACGACGAACGA (SEQ ID NO:810 ) |

| **Beacons** | | | | |
|---|---|---|---|---|
| MB_FUK_11670_1a | | CGACATGCCGCGTTAAAGAGTTC GTTTCGTCGAGCGCATGTCG (SEQ ID NO: 811) | | |
| MB_FUK_11670_1b | | CGACAGCCGCGTTAAAGAGTT CGTTTCGTCGAGCGCTGTCG (SEQ ID NO: 812) | | |
| PDCD4_11827 | B | CGACATGCCCGCAAATCCAAC CGCGCCCTGCATGTCG (SEQ ID NO: 813) | | |
| PHACTR3_11706 | B | CGACATGCCCGAACCCATAAC CGCGTCGAAGCATGTCG (SEQ ID NO: 814) | | |

Methylation specific real-time PCR is performed on either BioTrove (high throughput) or LC480 (Roche) in 384 format.

Sample UIDs are loaded into OMSpark™ software which randomizes the samples position according to sample type (C, N, NN). We always try to load the same number (n) of cancer and normal samples

### BioTrove OpenArrayTM Transcript Analysis System (BioTrove, Inc.12 Gill Street Suite 4000 Woburn, MA 01801-1728)

Each BioTrove array consist out of 48 subarrays each containing 64 through-holes. Per subarray 8 through-holes are used as negative control, 56 are spotted with primers at a concentration of 250nM. Each subarray is loaded with a mix of LC Faststart DNA SYBR Green I Master Mix (Roche), SYBR Green I (Sigma) end dilution 1:1, 6, Glycerol end concentration 0,5%, Pluronic F-68 (Gibco) end concentration 0,2%, BSA end concentration 1mg/ml, Roche MgC12 end concentration 1mM, Formamide end concentration 8% and 50ng BT-treated gDNA (calculated on the start concentration before BT-treatment). PCR specifications:90°C-10", (43°C-18", 49°C-60", 77°C-22", 72°C-70",95°C-28", 40 cycles),70°C-200", 45°C-5", melting curve 45°C>94°C (according to fixed BioTrove plate file)

### Analysis

### 1. PCR-O-Matic

Results are exported as csv file and imported in the PCR-O-Matic analyzing software which uses following algorithm to determine if there is a valuable amplification product formed.

Analyzing selected holes with Parameters from Light Cutoff (1000) (ID=29)

Min cycles until plateau=40, Min intensity difference=1000

Min dy/dx of intensity temp: 200

Min dy/dx of melting temperature: 200

1= amplification =Methylated

0= no amplification = Unmethylated

Because not all through holes are always filled we use the ROX (included in mastermix) signal to check the loading of the through holes. If the ROX signal is below a given value the results are marked as -1 by the software and discarded in the analysis to avoid false negatives. For each assay we calculate specificity and sensitivity as follows:

Sensitivity = Sum of methylated cancer samples/total count of cancer samples.

Specificity= 1- (Sum methylated normal samples/total count of normal samples)

Assays are ranked on specificity and sensitivity and the highest ranked assays are checked on the LC480 on a selection of "n" cancer and "n" normal patient samples.

### 2. Analysis based on melting peaks - Ct values.

A second more refined analysis is performed based on melting peaks which enables us to diffentiate between primer dimers and real amplification products, furthermore based on Ct values to be able to differentiate between methylated amplification products and background amplification from unmethylated material.

Minimal delta Ct = 5 ct's (assay dependent).

Again specificity and sensitivity are calculated and assays are ranked according to specificity and sensitivity. The highest ranked assays are used to develop building blocks.

### LightCycler LC480 (Roche)

In a 384 format we use 10µl PCR reaction mix per well which consists of LightCycler 480 SYBR Green I Master Mix, Roche MgCl₂ end concentration 1mM, BSA end concentration 1 mg/ml, Primer F end concentration 125 nM, Primer R end concentration 125 nM, and gDNA sample 10 ng/10 µl.

| **Cycling profile sybrgreen** | | | | | |
|---|---|---|---|---|---|
| | target temp | aquisition mode | hold | cycles | ramp rate |
| activation | 95 | none | 0:10:00 | 1 | 4,8 |
| amplifcation | 95 | | 0:00:10 | | 4,8 |
| | 60 | | 0:00:30 | | 2,5 |
| | 72 | | 0:00:01 | 45 | 4,8 |
| Melting curve | 95 | | 0:00:05 | | 4,8 |
| | 45 | | 0:01:00 | | 4,8 |
| | 95 | continous | | 1 | 2,5 |
| Cool down | 40 | | | 1 | 2 |

| Cycling profile Beacons | | | | | |
|---|---|---|---|---|---|
| | target temp | aquisition mode | hold | cycles | ramp rate |
| activation | 95 | none | 0:05:00 | 1 | 4,8 |
| amplification | 95 | | 0:00:30 | 45 | 4,8 |
| | 57 | aquisition mode | 0:00:30 | | 2,5 |
| | 72 | | 0:00:30 | | 4,8 |
| Melting curve | 95 | | 0:00:05 | 1 | 4,8 |
| | 45 | | 0:01:00 | | 2,5 |
| | 95 | continous | | | |

On the LightCycler we use the second analysis method based on melting peaks and Ct values.

### Results

For the investigation of the progression markers we used a BioTrove colon array which contains amongst others FUK_11670, PHACTR3_11706, and PDCD4_11827 assays.

We ran 37 cancer samples (Adenoma/Carcinoma), 11 normal samples and 27 normal normal samples on BioTrove and 40 cancer (Carcinoma) and 40 normal samples on LightCycler. The results obtained are summarized below. All three markers (FUK_11670, PHACTR3_11706, and PDCD4_11827) were differentially methylated compared to the normal samples.

| **BioTrove** | | | **LightCycler** | | |
|---|---|---|---|---|---|
| Assay | Specificity | Sensitivity | Assay | Specificity | Sensitivity |
| FUK_11670 | 84 | 58 | FUK_11670 | 92 | 50 |
| PHACTR3_11706 | 82 | 39 | PHACTR3_11706 | 87 | 56 |
| PDCD4_11827 | 92 | 53 | | | |

Methylation of the three markers was further investigated in 16 adenoma and 16 carcinoma samples. The results are listed below. Methylation of all three markers could be demonstrated in both adenoma's and carcinoma's. The three markers showed the same Ct average value for adenoma compared to carcinoma. These results indicate that all three markers are suitable for early detection of cancer. Our results indicated that PHACTR3 was the first marker out of the three markers to appear in adenoma samples.

| 16 Adenomas/16 Carcinomas samples | Average Ct value | | |
|---|---|---|---|
| | FUK_11670 | PDCD4 | PHACTR3 |
| average carcinoma's | 41.8 | 39.2 | 36.0 |
| average adenoma's | 41.6 | 38.5 | 34.5 |

**Table 1.**

| |
|---|
| NM_145059;Hs.7907;Homo sapiens L Homo sapiens fucokinase (FUK) |
| NM_183244;NM_080672;Hs.288513;Homo sapiens Q9H4T4 like (H17739), mRNA. |
| Homo sapiens phosphatase and actin regulator 3 (PHACTR3), transcript variant 2 |
| NM_145341;Hs.326248;Homo sapiens programmed cell death 4 (neoplastic transformation inhibitor) (PDCD4), transcript variant 2, mRNA. |
| NM_004669;Hs.64746;Homo sapiens chloride intracellular channel 3 (CLIC3), mRNA. |
| NM_000958;Hs.199248;Homo sapiens prostaglandin E receptor 4 (subtype EP4) (PTGER4), mRNA. |
| NM_032827;Hs.135569;Homo sapiens hypothetical protein FLJ14708 (FLJ14708), mRNA. |
| NM_006030;Hs.127436;Homo sapiens calcium channel, voltage-dependent, alpha 2/delta subunit 2 (CACNA2D2), mRNA. |
| NM_016569;Hs.267182;Homo sapiens T-box 3 (ulnar mammary syndrome) (TBX3), transcript variant 2, mRNA. |
| NM_001546;Hs.34853;Homo sapiens inhibitor of DNA binding 4, dominant negative helix-loop-helix protein (ID4), mRNA. |
| NM_001249;Hs.80975;Homo sapiens ectonucleoside triphosphate diphosphohydrolase 5 (ENTPD5), mRNA. |
| NM_014310;Hs.248222;Homo sapiens RASD family, member 2 (RASD2), mRNA. |
| NM_002731;Hs.87773;Homo sapiens protein kinase, cAMP-dependent, catalytic, beta (PRKACB), mRNA. |
| NM_005905;Hs.123119;Homo sapiens MAD, mothers against decapentaplegic homolog 9 (Drosophila) (MADH9), mRNA. |
| NM_012413;Hs.79033;Homo sapiens glutaminyl-peptide cyclotransferase (glutaminyl cyclase) (QPCT), mRNA. |
| NM_013943;Hs.25035;Homo sapiens chloride intracellular channel 4 (CLIC4), mRNA. |
| NM_002167;Hs,76884;Homo sapiens inhibitor of DNA binding 3, dominant negative helix-loop-helix protein (ID3), mRNA. |
| NM_021911;Hs.103998;Homo sapiens gamma-aminobutyric acid (GABA) A receptor, beta 2 (GABRB2), transcript variant 1, MRNA. |
| NM_003882;Hs.194680;Homo sapiens WNT1 inducible signaling pathway protein 1 (WISP1), transcript variant 1, mRNA. |
| BC029775;Hs.112554;Homo sapiens, hypothetical gene LOC127421, clone MGC:35394 IMAGE:5186268, mRNA, complete cds. |
| BC008502;Hs.342655;Homo sapiens, clone MGC:14841 IMAGE:4295121, mRNA, complete cds. |
| AB037842;Hs.117268;Homo sapiens mRNA for KIAA1421 protein, partial cds. |
| NM_003279;Hs.182421;Homo sapiens troponin C2, fast (TNNC2), mRNA. |
| BC035027;Hs.173048;Homo sapiens, clone IMAGE:4828469, mRNA. |
| NM_025087;Hs.288462;Homo sapiens hypothetical protein FLJ21511 (FLJ21511), mRNA. |
| NM_005904;Hs.100602;Homo sapiens MAD, mothers against decapentaplegic homolog 7 (Drosophila) (MADH7), mRNA. |
| NM_016308;Hs.11463;Homo sapiens UMP-CMP kinase (UMP-CMPK), mRNA. |
| BF683837;Hs.94499;602140129F1 NIH_MGC_46 Homo sapiens cDNA clone IMAGE:4301287 5', mRNA sequence. |
| NM_001343;Hs.81988;Homo sapiens disabled homolog 2, mitogen-responsive phosphoprotein (Drosophila) (DAB2), mRNA. |
| NM_005375;Hs.1334;Homo sapiens v-myb myeloblastosis Viral oncogene homolog (avian) (MYB), mRNA. |
| NM_138409;Hs.20953;Homo sapiens hypothetical protein BC010003 (LOC112609), mRNA. |
| NM_133367;Hs.239388;Homo sapiens chromosome 6 open reading frame 33 (C6orf33), mRNA. |
| NM_012326;Hs.172740;Homo sapiens microtubule-associated protein, RP/EB family, member 3 (MAPRE3), mRNA. |
| NM_014353;Hs.3797;Homo sapiens RAB26, member RAS oncogene family (RAB26), mRNA. |
| NM_024513;Hs.257267;Homo sapiens FYVE and coiled-coil domain containing 1 (FYCO1), mRNA. |
| NM_005476;Hs.5920;Homo sapiens UDP-N-acetylglucosamine-2-epimerase/N-acetylmannosamine kinase (GNE), mRNA. |
| NM_172127;-;Homo sapiens calcium/calmodulin-dependent protein kinase (CaM kinase) II delta (CAMK2D), transcript variant 1, mRNA. |
| AK091130;Hs.351270;Homo sapiens cDNA FLJ33811 fis, clone CTONG2002095. |
| NM_014729;Hs.184297;Homo sapiens thymus high mobility group box protein TOX (TOX), mRNA. |
| NM_004453;Hs.323468;Homo sapiens electron-transferring-flavoprotein dehydrogenase (ETFDH), nuclear gene encoding mitochondrial protein, mRNA. |
| NM_000196;Hs.1376;Homo sapiens hydroxysteroid (11-beta) dehydrogenase 2 (HSD11B2), mRNA. |
| NM_018690;Hs.200333;Homo sapiens apolipoprotein B48 receptor (APOB48R), mRNA. |
| NM_005144;Hs.272367;Homo sapiens hairless (HR), transcript variant 1, mRNA. |
| NM_018662;Hs.26985;Homo sapiens disrupted in schizophrenia 1 (DISC1), mRNA. |
| NM_002398;Hs.170177;Homo sapiens Mels1, myeloid ecotropic viral integration site 1 homolog (mouse) (MEIS1), mRNA. |
| NM_004915;Hs.10237;Homo sapiens ATP-binding cassette, sub-family G (WHITE), member 1 (ABCG1), transcript variant 1, mRNA. |
| NM_001752;Hs.395771;Homo sapiens catalase (CAT), mRNA. |
| NM_019000;Hs.82273;Homo sapiens hypothetical protein FLJ20152 (FLJ20152), mRNA. |
| NM_016353;Hs.5943;Homo sapiens zinc finger, DHHC domain containing 2 (ZDHHC2), mRNA. |
| NM_003645;Hs.11729;Homo sapiens solute carrier family 27 (fatty acid transporter), member 2 (SLC27A2), mRNA. |
| NM_023028;Hs.278581;Homo sapiens fibroblast growth factor receptor 2 (bacteria-expressed kinase, keratinocyte growth factor receptor, craniofacial dysostosis .1 |
| transcript variant 10, m |
| NM_017682;Hs.190222;Homo sapiens vitelliform macular dystrophy 2-like protein 1 (VMD2L1), mRNA. |
| NM_016400;Hs.300954;Homo sapiens Huntingtin interacting protein K (HYPK), mRNA. |
| NM_016357;Hs.10706;Homo sapiens epithelial protein lost in neoplasm beta (EPLIN), mRNA. |
| NM_152621;Hs.353519;Homo sapiens hypothetical protein MGC26963 (MGC26963), mRNA. |
| NM_011699;Hs.114556;Homo sapiens hypothetical protein FLJ20174 (FLJ20174), mRNA. |
| NM_138764;Hs.159428;Homo sapiens BCL2-associated X protein (BAX), transcript variant epsilon, m RNA. |
| NM_007283;Hs.6721;Homo sapiens monoglyceride lipase (MGLL), mRNA. |
| NM_006416;Hs.82921;Homo sapiens solute carrier family 35 (CMP-sialic acid transporter), member 1 (SLC35A1), mRNA. |
| NM_003839:Hs.114676;Homo sapiens tumor necrosis factor receptor superfamily, member 11a, activator of NFKB (TNFRSF11A), mRNA. |
| NM_000439;Hs.78977;Homo sapiens proprotein convertase subtilisinlkexin type 1 (PCSK1), mRNA. |
| NM_014899;Hs.10432;Homo sapiens Rho-related BTB domain containing 3 (RHOBTB3), mRNA. |
| NM_001405;Hs.158306;Homo sapiens ephrin-A2 (EFNA2), mRNA. |
| NM_000901;Hs.1790;Homo sapiens nuclear receptor subfamily 3, group C, member 2 (NR3C2), mRNA. |
| NM_003359;Hs.28309;Homo sapiens UDP-glucose dehydrogenase (UGDH), mRNA. |
| NM_030919;Hs.70704;Homo sapiens chromosome 20 open reading frame 129 (C20orf129), mRNA. |
| BC002877;Hs.154145;Homo sapiens, Similar to hypothetical protein FLJ11585, clone MGC:11258 IMAGE:3942160, mRNA, complete cds. |
| NM_018453;Hs.433269;Homo sapiens chromosome 14 open reading frame 11 (C14orf11), mRNA. |
| NM_025137;Hs.288872;Homo sapiens hypothetical protein FLJ21439 (FLJ21439), mRNA. |
| NM_004315;Hs.75811;Homo sapiens N-acylsphingosine amidohydrolase (acid ceramidase) 1 (ASAH1), mRNA. |
| NM_016341;Hs.6733;Homo sapiens phospholipase C, epsilon 1 (PLCE1), mRNA. |
| NM_018259;Hs.17283;Homo sapiens hypothetical protein FU10890 (FLJ10890), mRNA. |
| NM_000177;Hs.290070;Homo sapiens gelsolin (amyloidosis, Finnish type) (GSN), mRNA. |
| NM_005570;Hs.287912;Homo sapiens lectin, mannose-binding,1 (LMAN1), mRNA. |
| AK075564;Hs.27373;Homo sapiens cDNA PSEC0264 fis, clone NT2RP3002337. |
| NM_000633;Hs.79241;Homo sapiens B-cell CLUlymphoma 2 (BCL2), nuclear gene encoding mitochondrial protein, transcript variant alpha, mRNA. |
| NM_052904:Hs.45056;Homo sapiens KIAA1900 protein (KIAA1900), mRNA. |
| NM_032848;Hs.250820;Homo sapiens hypothetical protein FLJ14827 (FLJ14827), mRNA. |
| NM_005172;Hs.247685;Homo sapiens atonal homolog 1 (Drosophila) (ATOH1), mRNA. |
| NM_016014;Hs.380389;Homo sapiens CGI-67 protein (CGI-67), mRNA. |
| NM_152369;Hs.234101;Homo sapiens hypothetical protein MGC45474 (MGC45474), mRNA. |
| BC011002;Hs.372775;Homo sapiens, clone IMAGE:3946502, mRNA, partial cds. |
| NM_016614;Hs.46847;Homo sapiens TRAF and TNF receptor-associated protein (TTRAP), mRNA. |
| BC041626;-;Homo sapiens, clone MGC:52394 IMAGE:4554923, mRNA, complete cds. |
| NM_005154;Hs.152818;Homo sapiens ubiquitin specific protease 8 (USP8), mRNA. |
| NM_002840;Hs.75216;Homo sapiens protein tyrosine phosphatase, receptor type, F (PTPRF), transcript variant 1, mRNA. |
| NM_024312;Hs.7041;Homo sapiens MGC4170 protein (MGC4170), mRNA. |
| NM_005263;Hs.73172;Homo sapiens growth factor independent 1 (GFI1), mRNA. |
| NM_018473;Hs.9676;Homo sapiens uncharacterized hypothalamus protein HT012 (HT012), mRNA. |
| NM_001829;Hs.174139;Homo sapiens chloride channel 3 (CLCN3), mRNA. |
| NM_138340;Hs.13377;Homo sapiens abhydrolase domain containing 3 (ABHD3), mRNA. |
| NM_030574;Hs.172803;Homo sapiens START domain containing 5 (STARD5), mRNA. |
| NM_030790;Hs.23047;Homo sapiens T-cell immunomodulatory protein (CDA08), mRNA. |
| AB033070;Hs.194408;Homo sapiens mRNA for KIAA1244 protein, partial cds. |
| NM_138818;Hs.352153;Homo sapiens hypothetical protein BC019095 (LOC158471), mRNA. |
| NM_005585;Hs.153863;Homo sapiens MAD, mothers against decapentaplegic homolog 6 (Drosophila) (MADH6), mRNA. |
| NM_004872;Hs.11441;Homo sapiens chromosome 1 open reading frame 8 (C1orf8), mRNA. |
| BC041376;-;Homo sapiens, Similar to hypothetical protein 5830442F04, clone MGC:43895 IMAGE:5274634, mRNA, complete cds. |
| BC035149;Hs.247309;Homo sapiens, clone IMAGE:5264837, mRNA. |
| NM_020831;Hs.31146;Homo sapiens megakaryoblastic leukemia (translocation) 1 (MKL1), mRNA. |
| NM_032387;Hs.105448;Homo sapiens protein kinase, lysine deficient 4 (PRKWNK4), mRNA. |
| NM_001609;Hs.81934;Homo sapiens acyl-Coenzyme A dehydrogenase, short/branched chain (ACADSB), nuclear gene encoding mitochondrial protein, mRNA. |
| AB033025:Hs.50081;Homo sapiens mRNA for KIAA1199 protein, partial cds. |
| NM_152332;Hs.57787;Homo sapiens chromosome 14 open reading frame 47 (C14orf47), mRNA. |
| NM_000773;Hs.75183;Homo sapiens cytochrome P450, subfamily IIE (ethanol-inducible), polypeptide 1 (CYP2E1), mRNA. |
| NM_153498;Hs.279788;Homo sapiens CamKl-like protein kinase (CKLiK), transcript variant 2, mRNA. |
| NM_014047;Hs.420065;Homo sapiens HSPC023 protein (HSPC023), mRNA. |
| NM_022486;Hs.28564;Homo sapiens hypothetical protein DKFZp761E1824 (DKFZP761E1824), mRNA. |
| NM_006493;Hs.30213;Homo sapiens ceroid-lipofuscinosis, neuronal 5 (CLN5), mRNA. |
| NM_025163;Hs.289077;Homo sapiens hypothetical protein FLJ12768 (FLJ12768), mRNA. |
| NM_138448;Hs.422682;Homo sapiens acylphosphatase 2, muscle type (ACYP2), mRNA. |
| AB019210;Hs.26612;Homo sapiens BM32A mRNA for PMMLP, complete cds. |
| AF353674;Hs.7367;Homo sapiens BTB domain protein (BDPL) mRNA. partial cds. |
| NM_002979;Hs.75760;Homo sapiens sterol carrier protein 2 (SCP2), mRNA. |
| NM_005630:Hs.83974:Homo sapiens solute carrier family 21 (prostaglandin transporter), member 2 (SLC21A2), mRNA. |
| NM_004438;Hs.73964;Homo sapiens EphA4 (EPHA4), mRNA. |
| NM_000015;Hs.2;Homo sapiens N-acetyltransferase 2 (arylamine N-acetyltransferase) (NAT2), mRNA. |
| NM_003205;Hs.21704;Homo sapiens transcription factor 12 (HTF4, helix-loop-helix transcription factors 4) (TCF12), mRNA. |
| AL122070;-;Homo sapiens mRNA; cDNA DKFZp434E0535 (from clone DKFZp434E0535); partial cds. |
| NM_018281;Hs.34579;Homo sapiens hypothetical protein FLJ10948 (FLJ10948), mRNA. |
| NM_020403;Hs.12450;Homo sapiens protocadherin 9 (PCDH9), mRNA. |
| NM_002257;Hs.123107;Homo sapiens kallikrein 1, renal/pancreas/salivary (KLK1), inRNA. |
| NM_015141;Hs.82432;Homo sapiens KIAA0089 protein (KIAA0089), mRNA. |
| NM_018211;Hs.49933;Homo sapiens hypothetical protein FLJ10770 (KIAA1579), mRNA. |
| NM_152320;Hs.23492;Homo sapiens hypothetical protein FLJ31295 (FLJ31295), mRNA. |
| NM_031472;Hs.326586;Homo sapiens hypothetical protein MGC11134 (MGC11134), mRNA. |
| NM_005618;Hs.389889;Homo sapiens delta-like 1 (Drosophila) (DLL1), mRNA. |
| NM_012190;Hs.9520;Homo sapiens formyltetrahydrofolate dehydrogenase (FTHFD), transcript variant 1, mRNA. |
| NM_016651;Hs.489S0;Homo sapiens heptacellular carcinoma novel gene 3 (DAPPER1), mRNA. |
| NM_006796;Hs.29385;Homo sapiens AFG3 ATPase family gene 3-like 2 (yeast) (AFG3L2), nuclear gene encoding mitochondrial protein, mRNA. |
| NM_000983;Hs.326249;Homo sapiens ribosomal protein L22 (RPL22), mRNA. |
| NM_017423;Hs.246315;Homo sapiens UDP-N-acetyl-alpha-D-galactosam ine:polypeptide N-acetylgaiactosaminyltransferase 7 (GaiNAc-T7) (GALNT7), mRNA. |
| NM_003808;Hs.54673;Homo sapiens tumor necrosis factor (ligand) superfamily, member 13 (TNFSF13), transcript variant alpha, mRNA. |
| NM_004496;Hs.70604;Homo sapiens forkhead box A1 (FOXA1), mRNA. |
| NM_153446;Hs.374679;Homo sapiens beta 1,4 N-acetylgalactosaminyltransferase (B4GALT), mRNA. |
| NM_025212;Hs.118569;Homo sapiens Dvl-binding protein IDAX (inhibition of the Dvl and Axin complex) (IDAX), mRNA. |
| NM_005173:Hs.5541:Homo sapiens ATPase, Ca++ transporting, ubiquitous (ATP2A3), mRNA. |
| NM_005637;Hs.153221;Homo sapiens synovial sarcoma translocation, chromosome 18 (SS18), mRNA. |
| NM_020233;Hs.47668;Homo sapiens x 006 protein (MDS006), mRNA. |
| NM_006278;Hs.75268;Homo sapiens sialyltransferase 4C (beta-galactoside alpha-2,3-sialytransferase) (SIAT4C), mRNA. |
| NM_000293;Hs.78060;Homo sapiens phosphorylase kinase, beta (PHKB), mRNA. |
| NM_006197;Hs.75737;Homo sapiens pericentriolar material 1 (PCM1), mRNA. |
| NM_015111;AB002339;Hs.101761;Human mRNA for KIAA0341 gene, partial cds. |
| NM_001145;Hs.332764;Homo sapiens angiogenin, ribonuclease, RNase A family, 5 (ANG), mRNA. |
| NM_005080;Hs.149923;Homo sapiens X-box binding protein 1 (XBP1), mRNA. |
| NM_030912;Hs.54580;Homo sapiens tripartite motif-containing 8 (TRIM8), mRNA. |
| NM_025244;Hs.116116;Homo sapiens testis specific, 10 (TSGA10), mRNA. |
| NM_002165;Hs.75424;Homo sapiens inhibitor of DNA binding 1, dominant negative helix-loop-helix protein (ID1), mRNA. |
| NM_002828;Hs.82829;Homo sapiens protein tyrosine phosphatase, non-receptor type 2 (PTPN2), transcript variant 1, mRNA. |
| NM_013230;Hs.375108;Homo sapiens CD24 antigen (small cell lung carcinoma cluster 4 antigen) (CD24), mRNA. |
| NM_017707;Hs.44579;Homo sapiens up-regulated in liver cancer 1 (UPLC1), mRNA. |
| NM_138811;Hs.122055;Homo sapiens hypothetical protein BC015397 (LOC136895), mRNA. |
| NM_000690;Hs.195432;Homo sapiens aldehyde dehydrogenase 2 family (mitochondrial) (ALDH2), nuclear gene encoding mitochondrial protein, mRNA. |
| NM_006839;Hs.78504;Homo sapiens inner membrane protein, mitochondrial (mitofilin) (IMMT), mRNA. |
| NM_032270;Hs.193669;Homo sapiens hypothetical protein AD158 (AD158), mRNA. |
| NM_032857;Hs.374554;Homo sapiens lactamase, beta (LACTB), transcript variant 1, nuclear gene encoding mitochondrial protein, mRNA. |
| NM_005606;Hs.18069;Homo sapiens legumain (LGMN), mRNA. |
| NM_000520;Hs.119403;Homo sapiens hexosaminidase A (alpha polypeptide) (HEXA), mRNA. |
| NM_019012;Hs.86149;Homo sapiens phosphoinositol 3-phosphate-binding protein-2 (PEPP2), mRNA. |
| NM_145160;Hs.250870;Homo sapiens mltogen-activated protein kinase 5 (MAP2K5), transcript variant A, mRNA. |
| AK092094;Hs.166575;Homo sapiens cDNA FLJ34775 fis, clone NT2NE2003315. |
| NM_004634;Hs.1004;Homo sapiens bromodomain and PHD finger containing, 1 (BRPF1), mRNA. |
| NM_025181;Hs.108812;Homo sapiens hypothetical protein FLJ22004 (FLJ22004), mRNA. |
| NM_018421;Hs.135917;Homo sapiens TBC1 domain family, member 2 (TBC1D2), mRNA. |
| NM_139058;Hs.157208;Homo sapiens aristaless related homeobox (ARX), mRNA. |
| NM_018147;Hs.173438;Homo sapiens Fas apoptotic inhibitory molecule (FAIM), mRNA. |
| NM_013443;Hs.109672;Homo sapiens CMP-NeuAC:(beta)-N-acetylgalactosaminide (alpha)2,6-sialyltransferase member VI (ST6GALNAC6), mRNA. |
| NM_015392;Hs.105547;Homo sapiens neural proliferation, differentiation and control, 1 (NPDC1), mRNA. |
| BC022357;Hs.82202;Homo sapiens, clone MGC:23866 IMAGE:4297017, mRNA, complete cds. |
| BC030557;Hs.159066;Homo sapiens, clone MGC:40430 IMAGE:5218944, mRNA, complete cds. |
| NM_022154;Hs.284205;Homo sapiens BCG-induced gene in monocytes, clone 103 (BIGM103), mRNA. |
| NM_080672;Hs.288513;Homo sapiens Q9H4T4 like (H17739), mRNA. |
| NM_014142;Hs.301957;Homo sapiens nudix (nucleoside diphosphate linked moiety X)-type motif 5 (NUDT5), mRNA. |
| NM_021005;Hs.347991;Homo sapiens nuclear receptor subfamily 2, group F, member 2 (NR2F2)"mRNA. |
| BC040548;-;Homo sapiens, Similar to hypothetical protein MGC38960, clone IMAGE:5288206, mRNA. |
| NM_001749;Hs.74451;Homo sapiens calpain, small subunit 1 (CAPNS1), mRNA. |
| BC034751;Hs.115274;Homo sapiens, Indian hedgehog homolog (Drosophila), clone MGC:34815 IMAGE:5182642, mRNA, complete cds. |
| NM_006834;Hs.32217;Homo sapiens RAB32, member RAS oncogene family (RAB32), mRNA. |
| NM_033342;Hs.343661;Homo sapiens tripartite motif-containing 7 (TRIM7), mRNA. |
| NM_014172;Hs.297214;Homo sapiens phosphohistidine phosphatase (PHP14), mRNA. |
| BC019669;Hs.181165;Homo sapiens, eukaryotic translation elongation factor 1 alpha 1, clone MGC:25051 IMAGE:4478650, mRNA, complete cds. |
| NM_006377;Hs.155001;Homo sapiens unc-13-like (C. elegans) (UNC13), mRNA. |
| NM_017768;Hs.50848;Homo sapiens hypothetical protein FLJ20331 (FLJ20331), mRNA. |
| NM_015079;Hs.126084;Homo sapiens KIAA1055 protein (KIAA1055), mRNA. |
| NM_005340;Hs.256697;Homo sapiens histidine triad nucleotide binding protein 1 (HINT1), mRNA. |
| NM_022827;Hs.103147;Homo sapiens hypothetical protein FLJ21347 (FLJ21347), mRNA. |
| NM_052968;Hs.283923;Homo sapiens apolipoprotein A-V (APOA5), mRNA. |
| NM_033028;-;Homo sapiens Bardet-Biedl syndrome 4 (BBS4), mRNA. |
| NM_005923;Hs.151988;Homo sapiens mitogen-activated protein kinase kinase kinase 5 (MAP3K5), mRNA. |
| NM_001874;Hs.334873;Homo sapiens carboxypeptidase M (CPM), mRNA. |
| NM_152487;Hs.84522;Homo sapiens hypothetical protein FLJ31842 (FLJ31842), mRNA. |
| NM_004787;Hs.29802;Homo sapiens slit homolog 2 (Drosophila) (SLIT2), mRNA. |
| NM_153225;Hs.41185;Homo sapiens hypothetical protein FLJ40021 (FLJ40021), mRNA. |
| NM_004799;Hs.194716;Homo sapiens MAD, mothers against decapentaplegic homolog (Drosophila) interacting protein, receptor activation anchor (MADHIP), transaminase |
| NM_003383;Hs.73729;Homo sapiens very low density lipoprotein receptor (VLDLR), mRNA. |
| NM_000698;Hs.89499;Homo sapiens arachidonate 5-lipoxygenase (ALOX5), mRNA. |
| BC040191;Hs.192775;Homo sapiens, Similar to expressed sequence A1414849, clone IMAGE:4814106, mRNA. |
| NM_018043;Hs.26176;Homo sapiens hypothetical protein FLJ10261 (FLJ10261), mRNA. |
| NM_000112;Hs.29981;Homo sapiens solute carrier family 26 (sulfate transporter), member 2 (SLC26A2), mRNA. |
| , NM_005309;Hs.103502;Homo sapiens glutamic-pyruvate transaminase (alanine aminotransferase) (GPT), mRNA. |
| NM_004748;Hs.283753;Homo sapiens cell cycle progression 8 protein (CPR8), mRNA. |
| AB067471;Hs.345063;Homo sapiens mRNA for KIAA1884 protein, partial cds. |
| NM_004056;Hs.250502;Homo sapiens carbonic anhydrase VIII (CA8), mRNA. |
| NM_002760;Hs.56336;Homo sapiens protein kinase, Y-linked (PRKY), mRNA. |
| NM_006361;Hs.66731;Homo sapiens homeo box B13 (HOXB13), mRNA. |
| NM_015270;Hs.12373;Homo sapiens adenylate cyclase 6 (ADCY6), transcript variant 1. mRNA. |
| NM_001177;Hs.242894;Homo sapiens ADP-ribosylation factor-like 1 (ARL1), mRNA. |
| NM_013390;Hs.160417;Homo sapiens transmembrane protein 2 (TMEM2), mRNA. |
| NM_152429;Hs.37716;Homo sapiens hypothetical protein MGC39320 (MGC39320), mRNA. |
| NM_018668;Hs.26510;Homo sapiens vacuolar protein sorting 33B (yeast) (VPS33B), mRNA. |
| NM_016013;Hs.106529;Homo sapiens CGI-65 protein (CIA30), mRNA. |
| NM_003159;Hs.50905;Homo sapiens cyclin-dependent kinase-like 5 (CDKL5), mRNA. |
| NM_004232;Hs.44439;Homo sapiens suppressor of cytokine signaling 4 (SOCS4), mRNA. |
| BC035372;Hs.11067;Homo sapiens, clone MGC:35266 IMAGE:5174235, mRNA, complete cds. |
| NM_004869;Hs.126550;Homo sapiens vacuolar protein sorting 4B (yeast) (VPS4B), mRNA. |
| NM_000627;Hs.241257;Homo sapiens latent transforming growth factor beta binding protein 1 (LTBP1), mRNA. |
| NM_024081;Hs.306226;Homo sapiens transmembrane gamma-carboxyglutamic acid protein 4 (TMG4), mRNA. |
| NM_012421;Hs.13321;Homo sapiens rearranged L-myc fusion sequence (RLF), mRNA. |
| NM_004819;Hs.107019;Homo sapiens symplekin; Huntingfin interacting protein I (SPK), mRNA. |
| AF091236;Hs.384498;Homo sapiens ADP/ATP translocase mRNA, partial cds. |
| NM_031304;Hs.91103;Homo sapiens hypothetical protein MGC4293 (MGC4293), mRNA. |
| NM_017590;Hs.25347:Homo sapiens ubiquitous tetratricopeptide containing protein RoXaN (RoXaN), mRNA. |
| NM_033338;NM_033339;Hs.9216;Homo sapiens caspase 7, apoptosis-related cysteine protease (CASP7), transcript variant gamma, mRNA. |
| NM_018144;NM_018145;Hs.8055;Homo sapiens hypothetical protein FLJ10579 (FLJ10579), mRNA. |

The sequences of each of the genes is incorporated by reference herein as they existed in the public database on the date of filing of the subject application.

### CLAUSES:

1. A method for identifying colorectal cancer or predisposition to colorectal cancer, comprising:
   detecting in a test sample containing colorectal cells or nucleic acids from colorectal cells, epigenetic silencing of at least one gene listed in Table 1;
   identifying the test sample as neoplastic or predisposed to neoplasia or from cells that are neoplastic or predisposed to neoplasia.
2. The method of clause 1 wherein the test sample contains adenoma cells.
3. The method of clause 1 wherein the test sample contains carcinoma cells.
4. The method of clause 3 wherein the at least one gene is selected from the group consisting of NM_145059.1, NM_183244.1, NM_45341.2, NM_080672.3, NM_002630, NM_003383, NM_005504, NM_016269, NM_006988, NM_021637, NM_001888, and NM_014899; and wherein the test sample is identified as containing neoplastic cells or nucleic acids from neoplastic cells.
5. The method of clause 2 wherein the test sample contains adenoma cells and wherein the at least one gene is selected from the group consisting of NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899; and wherein the test sample is identified as containing cells which are likely to progress to carcinoma or as containing nucleic acids from said cells.
6. The method of clause 1 wherein the test sample is from a biopsy specimen.
7. The method of clause 1 wherein the test sample is from a surgical specimen.
8. The method of clause 1 wherein the test sample is from a cytological specimen.
9. The method of clause 1 wherein the test sample is isolated from stool, blood, or urine.
10. The method of clause 5 wherein surgical removal of neoplastic tissue is recommended to the patient.
11. The method of clause 5 wherein adjuvant chemotherapy is recommended to the patient.
12. The method of clause 5 wherein adjuvant radiation therapy is recommended to the patient.
13. The method of clause 5 wherein a colonoscopy is recommended to the patient.
14. The method of clause 5 wherein increased frequency of colonoscopy is recommended to the patient.
15. The method of clause 5 wherein an imaging study of the colon is recommended to the patient.
16. The method of clause 1 wherein epigenetic silencing of at least two genes is detected.
17. The method of clause 1 wherein epigenetic silencing of at least three genes is detected.
18. The method of clause 1 wherein epigenetic silencing is detected by detecting methylation of a CpG dinucleotide motif in the gene.
19. The method of clause 1 wherein epigenetic silencing is detected by detecting methylation of a CpG dinucleotide motif in a promoter of the gene.
20. The method of clause 1 wherein epigenetic silencing is detected by detecting diminished expression of mRNA of the gene.
21. The method of clause 1 wherein epigenetic silencing is detected by detecting diminished expression of protein encoded by the gene.
22. The method of clause 18 wherein methylation is detected by contacting at least a portion of the gene with a methylation-sensitive restriction endonuclease, said endonuclease preferentially cleaving methylated recognition sites relative to non-methylated recognition sites, whereby cleavage of the portion of the gene indicates methylation of the portion of the gene.
23. The method of clause 22 wherein the methylation-sensitive restriction endonuclease is selected from the group consisting of Acc III, Ban I, BstN I, Msp I, and Xma I.
24. The method of clause 18 wherein methylation is detected by contacting at least a portion of the gene with a methylation-sensitive restriction endonuclease, said endonuclease preferentially cleaving non-methylated recognition sites relative to methylated recognition sites, whereby cleavage of the portion of the gene indicates non-methylation of the portion of the gene provided that the gene comprises a recognition site for the methylation-sensitive restriction endonuclease.
25. The method of clause 24 wherein the methylation-sensitive restriction endonuclease is selected from the group consisting of Ace II, Ava I, BssH II, BstU I, Hpa II, and Not I.
26. The method of clause 18 wherein methylation is detected by:
   contacting at least a portion of the gene of the test cell with a chemical reagent that selectively modifies a non-methylated cytosine residue relative to a methylated cytosine residue, or selectively modifies a methylated cytosine residue relative to a non-methylated cytosine residue; and
   detecting a product generated due to said contacting.
27. The method of clause 26 wherein the step of detecting comprises amplification with at least one primer that hybridizes to a sequence comprising a modified non-methylated CpG dinucleotide motif but not to a sequence comprising an unmodified methylated CpG dinucleotide motif thereby forming amplification products.
28. The method of clause 26 wherein the step of detecting comprises amplification with at least one primer that hybridizes to a sequence comprising an unmodified methylated CpG dinucleotide motif but not to a sequence comprising a modified non-methylated CpG dinucleotide motif thereby forming amplification products.
29. The method of clause 27 wherein the amplification products are detected using (a) a first oligonucleotide probe which hybridizes to a sequence comprising a modified non-methylated CpG dinucleotide motif but not to a sequence comprising an unmodified methylated CpG dinucleotide motif, (b) a second oligonucleotide probe that hybridizes to a sequence comprising an unmodified methylated CpG dinucleotide motif but not to sequence comprising a modified non-methylated CpG dinucleotide motif, or (c) both said first and second oligonucleotide probes.
30. The method of clause 28 wherein the amplification products are detected using (a) a first oligonucleotide probe which hybridizes to a sequence comprising a modified non-methylated CpG dinucleotide motif but not to a sequence comprising an unmodified methylated CpG dinucleotide motif, (b) a second oligonucleotide probe that hybridizes to a sequence comprising an unmodified methylated CpG dinucleotide motif but not to sequence comprising a modified non-methylated CpG dinucleotide motif, or (c) both said first and second oligonucleotide probes.
31. The method of clause 26 wherein the product is detected by a method selected from the group consisting of electrophoresis, chromatography, and mass spectrometry.
32. The method of clause 26 wherein the chemical reagent is hydrazine.
33. The method of clause 32 further comprising cleavage of the hydrazine-contacted at least a portion of the gene with piped dine.
34. The method of clause 26 wherein the chemical reagent comprises bisulfite ions.
35. The method of clause 34 further comprising treating the bisulfite ion-contacted at least a portion of the gene with alkali.
36. The method of clause 18 wherein methylation is detected by:
   amplifying at least a portion of the gene, said portion comprising a CpG dinucleotide motif, to form amplification products;
   contacting the amplification products with a chemical reagent that selectively modifies a non-methylated cytosine residue relative to a methylated cytosine residue, or selectively modifies a methylated cytosine residue relative to a non-methylated cytosine residue; and
   detecting a product generated due to said contacting using (a) a first oligonucleotide probe which hybridizes to a sequence comprising a modified non-methylated CpG dinucleotide motif but not to a sequence comprising an unmodified methylated CpG dinucleotide motif, (b) a second oligonucleotide probe that hybridizes to a sequence comprising an unmodified methylated CpG dinucleotide motif but not to sequence comprising a modified non-methylated CpG dinucleotide motif, or (c) both said first and second oligonucleotide probes.
37. A method of reducing or inhibiting neoplastic growth of a cell which exhibits epigenetic silenced transcription of at least one gene associated with a cancer, the method comprising:
   determining that a cell has an epigenetic silenced gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899;
   restoring expression of a polypeptide encoded by the epigenetic silenced gene in the cell by contacting the cell with a CpG dinucleotide demethylating agent, thereby reducing or inhibiting unregulated growth of the cell.
38. The method of clause 37 wherein the contacting is performed in vitro.
39. The method of clause 37 wherein the contacting is performed in vivo by administering the agent to a mammalian subject comprising the cell.
40. The method of clause 37 wherein the demethylating agent is selected from the group consisting of 5-aza-2'-deoxycytidine, 5-aza-cytidine, Zebularine, procaine, and L- ethionine.
41. A method of reducing or inhibiting neoplastic growth of a cell which exhibits epigenetic silenced transcription of at least one gene associated with a cancer, the method comprising:
   determining that a cell has an epigenetic silenced gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899;
   introducing a polynucleotide encoding a polypeptide into the cell, wherein the polypeptide is encoded by said gene, wherein the polypeptide is expressed in the cell thereby restoring expression of the polypeptide in the cell.
42. A method of treating a cancer patient, the method comprising:
   determining that a cancer cell in the patient has an epigenetic silenced gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899;
   administering a demethylating agent to the patient in sufficient amounts to restore expression of the epigenetic silenced gene in the patient's cancer cells.
43. The method of clause 42 wherein the demethylating agent is selected from the group consisting of 5-aza-2'-deoxycytidine, 5-aza-cytidine, Zebularine, procaine, and L- ethionine.
44. A method of treating a cancer patient, the method comprising:
   determining that a cancer cell in the patient has an epigenetic silenced gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899;
   administering to the patient a polynucleotide encoding a polypeptide, wherein the polypeptide is encoded by the epigenetic silenced gene, wherein the polypeptide is expressed in the patient's tumor thereby restoring expression of the polypeptide in the cancer.
45. A method for selecting a therapeutic strategy for treating a cancer patient, comprising:
   identifying a gene whose expression in cancer cells of the patient is reactivated by a demethylating agent, wherein the gene is selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM-001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899; and
   selecting a therapeutic agent which increases expression of the gene for treating said cancer patient.
46. The method of clause 45 further comprising the step of prescribing the therapeutic agent for said cancer patient.
47. The method of clause 45 further comprising the step of administering the therapeutic agent to said cancer patient.
48. The method of clause 45 wherein the therapeutic agent comprises a polynucleotide encoding the gene.
49. The method of clause 45 wherein the demethylating agent is 5-aza-2'-deoxycytidine.
50. The method of clause 45 wherein the therapeutic agent is 5-aza-2'-deoxycytidine.
51. The method of clause 45 wherein the cancer cells are obtained from a surgical specimen.
52. The method of clause 45 wherein the cancer cells are obtained from a biopsy specimen.
53. The method of clause 45 wherein the cancer cells are obtained from a cytological sample.
54. The method of clause 45 wherein the cancer cells are obtained from stool, blood, or urine.
55. A kit for assessing methylation in a test sample, comprising in a package:
   a reagent that (a) modifies methylated cytosine residues but not non-methylated cytosine residues, or that (b); modifies non-methylated cytosine residues but not methylated cytosine residues; and
   a pair of oligonucleotide primers that specifically hybridizes under amplification conditions to a region of a gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and
   NM_002899, wherein the region is within about 1kb of said gene's transcription start site.
56. The kit of clause 55 wherein at least one of said pair of oligonucleotide primers hybridizes to a sequence comprising a modified non-methylated CpG dinucleotide motif but not to a sequence comprising an unmodified methylated CpG dinucleotide motif or wherein at least one of said pair of oligonucleotide primers hybridizes to a sequence comprising an unmodified methylated CpG dinucleotide motif but not to sequence comprising a modified non-methylated CpG dinucleotide motif.
57. The kit of clause 55 further comprising (a) a first oligonucleotide probe which hybridizes to a sequence comprising a modified non-methylated CpG dinucleotide motif but not to a sequence comprising an unmodified methylated CpG dinucleotide motif, (b) a second oligonucleotide probe that hybridizes to a sequence comprising an unmodified methylated CpG dinucleotide motif but not to sequence comprising a modified non-methylated CpG dinucleotide motif, or (c) both said first and second oligonucleotide probes.
58. The kit of clause 56 further comprising (a) a first oligonucleotide probe which hybridizes to a sequence comprising a modified non-methylated CpG dinucleotide motif but not to a sequence comprising an unmodified methylated CpG dinucleotide motif, (b) a second oligonucleotide probe that hybridizes to a sequence comprising an unmodified methylated CpG dinucleotide motif but not to sequence comprising a modified non-methylated CpG dinucleotide motif, or (c) both said first and second oligonucleotide probes.
59. The kit of clause 55 further comprising an oligonucleotide probe.
60. The kit of clause 55 further comprising a DNA polymerase for amplifying DNA.

## Claims

1. A method for identifying colorectal cancer or predisposition to colorectal cancer, comprising:
detecting in a test sample containing colorectal cells or nucleic acids from colorectal cells, epigenetic silencing of the following gene NM_183244.1 (PHACTR3);
identifying the test sample as neoplastic or predisposed to neoplasia or from cells that are neoplastic or predisposed to neoplasia.

2. A method for identifying colorectal cancer or predisposition to colorectal cancer, comprising:
detecting in a test sample containing colorectal cells or nucleic acids from colorectal cells, epigenetic silencing of at least one gene listed in Table 1;
identifying the test sample as neoplastic or predisposed to neoplasia or from cells that are neoplastic or predisposed to neoplasia.

3. The method of claim 1 or claim 2 wherein the test sample contains adenoma cells or carcinoma cells.

4. The method of claim 2 wherein the test sample contains carcinoma cells and the at least one gene is selected from the group consisting of NM_145059.1, NM_183244.1, NM_45341.2, NM_080672.3, NM_002630, NM_003383, NM_005504, NM_016269, NM_006988, NM_021637, NM_001888, and NM_014899; and wherein the test sample is identified as containing neoplastic cells or nucleic acids from neoplastic cells.

5. The method of claim 2 wherein the test sample contains adenoma cells and wherein the at least one gene is selected from the group consisting of NM_145341, NM_001752, NM_014142, NM_001145, NM_016013, NM_017590, NM_152429, NM_138340, NM_052968, NM_183244, NM_199423, NM_015111, NM_002181, TTM_005637, NM_030790, NM_018144, NM_004232, NM_030912, NM_145059, NM_033338, NM_006834, NM_003014, NM_001343, NM_000963, NM_004385, and NM_002899; and
wherein the test sample is identified as containing cells which are likely to progress to carcinoma or as containing nucleic acids from said cells.

6. The method of claim 1 or claim 2 wherein the test sample is from a biopsy specimen, a surgical specimen, a cytological specimen, or is isolated from stool, blood, or urine.

7. The method of claim 5 wherein one of the following is recommended to the patient:
(1) surgical removal of neoplastic tissue;
(2) adjuvant chemotherapy;
(3) adjuvant radiation therapy;
(4) a colonoscopy;
(5) increased frequency of colonoscopy; and
(6) an imaging study of the colon.

8. The method of claim 2 wherein epigenetic silencing of at least two genes, more preferably at least three genes, is detected.

9. The method of claim 1 or claim 2 wherein epigenetic silencing is detected by detecting:
(1) methylation of a CpG dinucleotide motif in the gene;
(2) methylation of a CpG dinucleotide motif in a promoter of the gene;
(3) diminished expression of mRNA of the gene; or
(4) diminished expression of protein encoded by the gene.

10. Use of a CpG dinucleotide demethylating agent in the manufacture of a medicament for reducing or inhibiting neoplastic growth of a cell which exhibits epigenetic silenced transcription of the following gene: NM_183244.1 (PHACTR3).

11. Use of a CpG dinucleotide demethylating agent in the manufacture of a medicament for reducing or inhibiting neoplastic growth of a cell which exhibits epigenetic silenced transcription of a gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899.

12. An *in vitro* method of reducing or inhibiting neoplastic growth of a cell which exhibits epigenetic silenced transcription of at least one gene associated with a cancer, the method comprising:
determining that a cell has an epigenetic silenced NM_183244.1 (PHACTR3) gene;
restoring expression of a polypeptide encoded by the epigenetic silenced gene in the cell by contacting the cell with a CpG dinucleotide demethylating agent, thereby reducing or inhibiting unregulated growth of the cell.

13. An *in vitro* method of reducing or inhibiting neoplastic growth of a cell which exhibits epigenetic silenced transcription of at least one gene associated with a cancer, the method comprising:
determining that a cell has an epigenetic silenced gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899;
restoring expression of a polypeptide encoded by the epigenetic silenced gene in the cell by contacting the cell with a CpG dinucleotide demethylating agent, thereby reducing or inhibiting unregulated growth of the cell.

14. The use of claim 10 or claim 11, or the method of claim 12 or claim 13 wherein the demethylating agent is selected from the group consisting of 5-aza-2'-deoxycytidine, 5-aza-cytidine, Zebularine, procaine, and L- ethionine.

15. Use of a polynucleotide encoding a polypeptide in the manufacture of a medicament for treating a cancer patient,
wherein the patient has an epigenetic silenced NM_183244.1 (PHACTR3) gene;
wherein the polypeptide is encoded by the epigenetic silenced gene.

16. Use of a polynucleotide encoding a polypeptide in the manufacture of a medicament for treating a cancer patient,
wherein the patient has an epigenetic silenced gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899;
wherein the polypeptide is encoded by the epigenetic silenced gene.

17. A kit for assessing methylation in a test sample, comprising in a package:
a reagent that (a) modifies methylated cytosine residues but not non-methylated cytosine residues, or that (b); modifies non-methylated cytosine residues but not methylated cytosine residues; and
a pair of oligonucleotide primers that specifically hybridizes under amplification conditions to a region of the NM_183244.1 (PHACTR3) gene, wherein at least one of said pair of oligonucleotide primers hybridizes to a sequence comprising a modified non-methylated CpG dinucleotide motif but not to a sequence comprising an unmodified methylated CpG dinucleotide motif or wherein at least one of said pair of oligonucleotide primers hybridizes to a sequence comprising an unmodified methylated CpG dinucleotide motif but not to sequence comprising a modified non-methylated CpG dinucleotide motif.

18. A kit for assessing methylation in a test sample, comprising in a package:
a reagent that (a) modifies methylated cytosine residues but not non-methylated cytosine residues, or that (b); modifies non-methylated cytosine residues but not methylated cytosine residues; and
a pair of oligonucleotide primers that specifically hybridizes under amplification conditions to a region of a gene selected from the group consisting of NM_145059.1, NM_183244.1, NM_145341.2, NM_080672.3, NM_002630, NM_003383, NM_005504, NM_016269, NM_006988, NM_021637, NM_001888, NM_014899, NM_145341, NM_001752, NM_014142, NM_001145, NM_016013, NM_017590, NM_152429, NM_138340, NM_052968, NM_183244, NM_199423, NM_015111, NM_002181, NM_005637, NM_030790, NM_018144, NM_004232, NM_030912, NM_145059, NM_033338, NM_006834, NM_003014, NM_001343, NM_000963, NM_004385, and NM_002899, wherein at least one of said pair of oligonucleotide primers hybridizes to a sequence comprising a modified non-methylated CpG dinucleotide motif but not to a sequence comprising an unmodified methylated CpG dinucleotide motif or wherein at least one of said pair of oligonucleotide primers hybridizes to a sequence comprising an unmodified methylated CpG dinucleotide motif but not to sequence comprising a modified non-methylated CpG dinucleotide motif.
